(19) **Europäisches Patentamt / European Patent Office / Office européen des brevets**

(11) **EP 3 909 607 A1**

(12) # EUROPEAN PATENT APPLICATION
published in accordance with Art. 153(4) EPC

(43) Date of publication:
**17.11.2021 Bulletin 2021/46**

(21) Application number: **20738071.8**

(22) Date of filing: **13.01.2020**

(51) Int Cl.:
*A61K 39/395* (2006.01)  *A61K 47/02* (2006.01)
*A61K 47/22* (2006.01)  *A61K 47/14* (2017.01)
*A61K 47/26* (2006.01)  *A61P 19/02* (2006.01)
*A61P 29/00* (2006.01)  *C07K 16/24* (2006.01)
*A61K 39/00* (2006.01)

(86) International application number:
**PCT/KR2020/000611**

(87) International publication number:
**WO 2020/145789 (16.07.2020 Gazette 2020/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.01.2019 US 201962791541 P**

(71) Applicant: **Samsung Bioepis Co., Ltd.**
**Yeonsu-gu**
**Incheon 21987 (KR)**

(72) Inventors:
• **KIM, Mi-Gyeong**
  **Incheon 21987 (KR)**
• **LEE, Seung-ha**
  **Incheon 21987 (KR)**
• **JUNG, Youngseok**
  **Incheon 21987 (KR)**
• **HONG, Jahye**
  **Incheon 21987 (KR)**
• **JUNG, Soyun**
  **Incheon 21987 (KR)**
• **JOO, Kyung Hee**
  **Incheon 21987 (KR)**

• **NAM, Myungjoo**
  **Incheon 21987 (KR)**
• **JANG, Harah**
  **Incheon 21987 (KR)**
• **OH, Inyoung**
  **Incheon 21987 (KR)**
• **KIM, Inae**
  **Incheon 21987 (KR)**
• **LEE, Hun Joo**
  **Incheon 21987 (KR)**
• **KIM, Yongkook**
  **Incheon 21987 (KR)**
• **KIM, Han-soo**
  **Incheon 21987 (KR)**
• **JEONG, Woojin**
  **Incheon 21987 (KR)**
• **LEE, Nayoung**
  **Incheon 21987 (KR)**
• **LEE, Kyoungjin**
  **Incheon 21987 (KR)**
• **PARK, Jaewoo**
  **Incheon 21987 (KR)**

(74) Representative: **Boult Wade Tennant LLP**
**Salisbury Square House**
**8 Salisbury Square**
**London EC4Y 8AP (GB)**

(54) **PHARMACEUTICAL COMPOSITION COMPRISING ANTIBODY, DEVICE COMPRISING SAME, AND USE THEREOF**

(57)    The present invention provides a liquid pharmaceutical composition with improved protein stability and, more specifically, a stable liquid pharmaceutical composition comprising an antibody, and use thereof.

EP 3 909 607 A1

【FIG. 4】

**Description**

TECHNICAL FIELD

The present disclosure relates to a liquid pharmaceutical composition having improved protein stability, and more specifically, a stable liquid pharmaceutical composition comprising an antibody, and use thereof.

DESCRIPTION OF RELATED ART

Since antibody drugs have a higher molecular weight and a more complex structure, as compared with other general protein drugs, there are problems of physical and chemical instability. Instability of antibodies may be caused by various factors such as temperature, pH, buffer type, concentration, excipient, etc. The instability of antibodies may reduce activity of antibody drugs or may increase antigen immunogenicity, when administered to a human body. To develop a formulation that reduces the instability of antibody drugs and has improved quality, various methods, such as modification of a buffer solution, pH optimization, addition of a stabilizer, etc., have been employed.

For example, Humira® (adalimumab), which is a therapeutic agent for rheumatoid arthritis, is a liquid antibody drug at pH 5.2 containing 100 mg/mL of adalimumab, 4.2% mannitol, and 0.1% polysorbate 80, and free of a buffer.

There is a continuous need for the development of stable antibody formulations with minimal side effects.

CONTENT OF DISCLOSURE

SOLUTION TO PROBLEM

Accordingly, provided are an aqueous liquid composition for enhancing stability of a protein drug, specifically, a pharmaceutical composition including a protein drug, and use thereof.

An embodiment provides a pharmaceutical composition including

40 mg/ml to 200 mg/ml of adalimumab;
a buffer including phosphate; and
Histidine;
wherein pH is 5 to 7.

The pharmaceutical composition may be free of citrate, a pharmaceutically acceptable salt thereof, or both of them.

Further, the pharmaceutical composition may be free of salts other than pharmaceutically acceptable salts of phosphate, succinate, and acetate.

The pharmaceutical composition may be free of amino acids other than histidine.

The pharmaceutical composition may be free of ethylenediaminetetraacetic acid (EDTA).

The buffer may further include one or more selected from the group consisting of succinate and acetate.

When exposed to light, the pharmaceutical composition may have one or more of the following properties:

a variation ($\Delta\%Met_{256}=\%Met_{256/Light\text{-}exposured}—\%Met_{256/Base}$) in an oxidation rate ($\%Met_{256/light\text{-}exposured}$) of methionine, which is an amino acid residue at position 256 of adalimumab, is 68 % or less;
a variation ($\Delta\%HMW =\%HMW_{Light\text{-}exposured}—\%HMW_{Base}$) in a protein aggregation rate ($\%HMW$; % High Molecular Weight) is 16% or less, as measured by SE-HPLC; and
a variation ($\Delta\%LMW =\%LMW_{Light\text{-}exposured}—\%LMW_{Base}$) in a protein degradation rate ($\%LMW$; % Low Molecular Weight) is 1.6 % or less, as measured by SE-HPLC.

The pharmaceutical composition may further include a polyol.

The pharmaceutical composition may further include a surfactant. The surfactant may be polysorbates, for example, polysorbate 20.

Another embodiment provides a pharmaceutical composition for treating a disease, the pharmaceutical composition including the above-described pharmaceutical composition. The disease may be a disease in which adalimumab, which is an anti-TNFa antibody, exhibits therapeutic, alleviative, and/or prophylactic effects.

Still another embodiment provides a device including the pharmaceutical composition.

Still another embodiment provides a method of treating a disease, the method including administering the pharmaceutical composition to a subject in need of administration of adalimumab.

SOLUTION TO PROBLEM

The present disclosure provides an aqueous liquid composition for enhancing stability of a protein drug, specifically, a liquid pharmaceutical composition including an antibody. The aqueous liquid composition (liquid pharmaceutical composition) may include components described below, and a residual amount of aqueous medium (water (purified water), physiological saline, sterile water for injection, etc.).

Definitions

As used herein, the term "about" or "approximately" may be generally interpreted as including a value or range within ±20%, ±10%, ±5%, ±4%, ±3%, ±2%, or ±1 % of a given value or range.

The term "long-term storage stability" or "long-term stability" may mean that a pharmaceutical composition may be stored in a stable state for three months or more, six months or more, one year or more, or two years or more. For example, the term "storage" may be understood as a concept that embraces storage of a pharmaceutical composition under stress conditions, such as storage at 2 °C to 8 °C in a liquid-phase, frozen at -20 °C or lower, or subjected to one or more freeze-thaw cycles.

The term "stable state" may be understood as a state wherein an antibody (e.g., adalimumab) included in a pharmaceutical composition exhibits loss in its biological activity and/or structural stability (e.g., aggregation, degradation, denaturation (acidic or basic), oxidation, etc.) during the period of storage by 20% or less, 15% or less, 10% or less, or 5% or less, as compared with that of the initial storage.

The term "free of A" or "substantially free of A" may be understood to mean that A is completely absent or exists in a small amount without any substantial effect on properties of the composition. When the amount of A is not mentioned, it may be understood to mean an "undetectable amount".

The term "pharmaceutical composition" may refer to a preparation which is in such a form as to permit biological activities of active ingredients to be effective, and which includes no additional components significantly toxic to subjects to which the composition would be administered. The composition may be sterile.

The term "pharmaceutically acceptable" may refer to excipients, carriers, vehicles, diluents, additives, salts, etc., which are suitable for administration to a subject.

The term "pharmaceutical formulation" or "formulation" may refer to a product of a process in which an active drug is combined with chemical substances to produce a final medicinal product, wherein the final formulation refers to medicinal products, e.g., an injectable formulation, a capsule, a pill, a tablet, an emulsion, etc.

(1) Antibody

In the present disclosure, the antibody may refer to an anti-TNFa antibody. The anti-TNFa antibody may refer to all antibodies capable of binding to TNFα and regulating biological activities thereof.

Tumor necrosis factor-alpha (TNF-alpha, TNFα) is a cytokine produced by various kinds of cells, such as monocytes and macrophages, by stimulation with endotoxin, etc. TNFα, which activates TNF receptors to induce reactions such as T-cell activation, thymocyte proliferation, etc., is a key mediator of major inflammatory, immunological, and pathophysiological responses.

The anti-TNFa antibody may be in the form of a full-length antibody or an antibody fragment including an antigen-binding site thereof, but is not limited thereto. Specifically, the anti-TNFa antibody may be a human immunoglobulin G1 (hIgG1) monoclonal antibody, and more specifically, may be adalimumab.

Adalimumab is the first intact human antibody developed as a drug and was derived from a phage display technique, with the enhanced affinity thereof resulting from a modification in CDR. Adalimumab, also called D2E7, consists of 1330 amino acids with a molecular weight of about 148 kD. Adalimumab has been commercially available under the brand name of HUMIRA. HUMIRA has been approved for sale as a therapeutic agent for rheumatoid arthritis and applied for the treatment of Crohn's disease, ankylosing spondylitis, psoriatic arthritis, ulcerative colitis, plaque psoriasis, rheumatoid arthritis, polyarticular and juvenile idiopathic arthritis, etc. For more detailed information on adalimumab, a person skilled in the art could easily obtain the information from well-known database.

As used herein, the term "adalimumab" may also be interpreted as including adalimumab that is modified in the amino acid structure (deletion, addition, and/or substitution of amino acids) and/or in glycosylation property within a range that does not affect polypeptide functions.

In the pharmaceutical composition provided in the present disclosure, the anti-TNFa antibody may be included in a therapeutically effective amount. Specifically, the therapeutically effective amount may be about 25 mg/ml to about 200 mg/ml, about 25 mg/ml to about 175 mg /ml, about 25 mg/ml to about 150 mg/ml, about 25 mg/ml to about 125 mg/ml, about 25 mg/ml to about 100 mg/ml, about 25 mg/ml to about 75 mg/ml, about 25 mg/ml to about 50 mg/ml, about 30 mg/ml to about 200 mg/ml, about 30 mg/ml to about 175 mg/ml, about 30 mg/ml to about 150 mg/ml, about 30 mg/ml to about 125 mg/ml, about 30 mg/ml to about 100 mg/ml, about 30 mg/ml to about 75 mg/ml, about 30 mg/ml to about 50 mg/ml, about 40 mg/ml to about 200 mg/ml, about 40 mg/ml to about 175 mg /ml, about 40 mg/ml to about 150 mg/ml, about 40 mg/ml to about 125 mg/ml, about 40 mg/ml to about 100 mg/ml, about 40 mg/ml to about 75 mg/ml, about 40 mg/ml to about 50 mg/ml, about 50 mg/ml to about 200 mg/ml, about 50 mg/ml to about 175 mg /ml, about 50 mg/ml to about 150 mg/ml, about 50 mg/ml to about 125 mg/ml, about 50 mg/ml to about 100 mg/ml, about 75 mg/ml to about 200 mg/ml, about 75 mg/ml to about 175 mg/ml, about 75 mg/ml to about 150 mg/ml, about 75 mg/ml to about 125 mg/ml, about 75 mg/ml to about 100 mg/ml, about 100 mg/ml to about 200 mg/ml, about 100 mg/ml to about 175 mg/ml, about 100 mg/ml to 150 mg/ml, about 100 mg/ml to about 125 mg/ml, about 150 mg/ml to about 200 mg/ml, about 150 mg/ml to about 175 mg/ml, about 50 mg/ml, about 100 mg/ml, or about 200 mg/ml.

(2) Buffer

In the present disclosure, a buffer functions to adjust pH of the formulation, and may be an aqueous solution including phosphate or further including acetate, succinate, or both thereof in addition to phosphate.

In the present disclosure, phosphate, acetate, and succinate may also be interpreted as including a pharmaceutically acceptable salt thereof and/or a hydrate (e.g., monohydrate, etc.) thereof. The pharmaceutically acceptable salt may be one or more selected from the group consisting of a sodium salt, a potassium salt, a succinate salt, a phosphate salt, etc., but is not limited thereto.

Unless specified otherwise, the term "phosphate" may be interpreted as meaning one or more selected from the group consisting of phosphate, a pharmaceutically acceptable salt (e.g., sodium salt) thereof, and a hydrate thereof.

Unless specified otherwise, the term "succinate" may be interpreted as meaning one or more selected from the group consisting of succinate, a pharmaceutically acceptable salt (e.g., sodium salt) thereof, and a hydrate thereof.

Unless specified otherwise, the term "acetate" may be interpreted as meaning one or more selected from the group consisting of acetate, a pharmaceutically acceptable salt (e.g., sodium salt) thereof, and a hydrate thereof.

To improve stability of the anti-TNF alpha antibody included in the pharmaceutical composition provided in the present disclosure, pH of the buffer may be about 4.0 to about 8.0, and pH thereof may be about 4.0 to about 7.5, about 4.0 to about 7, about 4.0 to about 6.5, about 4.0 to about 6, about 4.0 to about 5.5, about 4.5 to about 7.5, about 4.5 to about 7, about 4.5 to about 6.5, about 4.5 to about 6, about 4.5 to about 5.5, about 4.8 to about 7.5, about 4.8 to about 7, about 4.8 to about 6.5, about 4.8 to about 6, about 4.8 to about 5.6, about 5.0 to about 7.5, about 5.0 to about 7, about 5.0 to about 6.5, about 5.0 to about 6, about 5.0 to about 5.5, about 5.2 to about 7.5, about 5.2 to about 7.2, about 5.2 to about 7, about 5.2 to about 6.8, about 5.2 to about 6.6, about 5.2 to about 6.5, about 5.5 to about 7.5, about 5.5 to about 7.2, about 5.5 to about 7, about 5.5 to about 6.8, about 5.5 to about 6.6, about 5.5 to about 6.5, about 6.0 to about 7.5, about 6.0 to about 7.2, about 6.0 to about 7, about 6.0 to about 6.8, about 6.0 to about 6.6, about 6.0 to about 6.5, about 6.4 to about 7.5, about 6.4 to about 7.2, about 6.4 to about 7, about 6.4 to about 6.8, about 6.4 to about 6.6, about 6.4 to about 6.5, about 5.2, about 5.3, about 5.4, or about 6.5.

The buffer may be included at a concentration of about 0.1 mM to about 50 mM, about 0.1 mM to about 40 mM, about 0.1 mM to about 30 mM, about 0.1 mM to about 20 mM, about 0.1 mM to about 13 mM, about 0.1 mM to about 10 mM, about 0.1 mM to about 7.8 mM, about 0.1 mM to about 6 mM, about 0.1 mM to about 4 mM, about 0.1 mM to about 3 mM, about 0.1 mM to about 2.7 mM, about 0.5 mM to about 50 mM, about 0.5 mM to about 40 mM, about 0.5 mM to about 30 mM, about 0.5 mM to about 20 mM, about 0.5 mM to about 13 mM, about 0.5 mM to about 10 mM, about 0.5 mM to about 7.8 mM, about 0.5 mM to about 6 mM, about 0.5 mM to about 4 mM, about 0.5 mM to about 3 mM, about 0.5 mM to about 2.7 mM, about 1 mM to about 50 mM, about 1 mM to about 40 mM, about 1 mM to about 30 mM, about 1 mM to about 20 mM, about 1 mM to about 13 mM, about 1 mM to about 10 mM, about 1 mM to about 7.8 mM, about 1 mM to about 6 mM, about 1 mM to about 5mM, about 1 mM to about 4 mM, about 1 mM to about 3 mM, about 1 mM to about 2.7 mM, about 1.7 mM to about 50 mM, about 1.7 mM to about 40 mM, about 1.7 mM to about 30 mM, about 1.7 mM to about 20 mM, about 1.7 mM to about 13 mM, about 1.7 mM to about 10 mM, about 1.7 mM to about 7.8 mM, about 1.7 mM to about 6 mM, about 1.7 mM to about 5mM, about 1.7 mM to about 4 mM, about 1.7 mM to about 3 mM, about 1.7 mM to about 2.7 mM, about 2 mM to about 50 mM, about 2 mM to about 40 mM, about 2 mM to about 30 mM, about 2 mM to about 20 mM, about 2 mM to about 13 mM, about 2 mM to about 10 mM, about 2 mM to about 7.8 mM, about 2 mM to about 6 mM, about 2 mM to about 3mM, about 2 mM to about 2.7 mM, about 2.5 mM to about 50 mM, about 2.5 mM to about 40 mM, about 2.5 mM to about 30 mM, about 2.5 mM to about 20 mM, about 2.5 mM to about 13 mM, about 2.5 mM to about 10 mM, about 2.5 mM to about 7.8 mM, about 2.5 mM to about 6 mM, about 2.7 mM to about 50 mM, about 2.7 mM to about 40 mM, about 2.7 mM to about 30 mM, about 2.7 mM to about 20 mM, about 2.7 mM to about 13 mM, about 2.7 mM to about 10 mM, about 2.7 mM to about 7.8 mM, about 2.7 mM to about 6 mM, about 2.7 mM to about 5.4 mM, about 3 mM to about 50 mM, about 3 mM to about 40 mM, about 3 mM to about 30 mM, about 3 mM to about 20 mM, about 3 mM to about 12 mM, about 3 mM to about 10 mM, about 3 mM to about 7.8 mM, about 3 mM to about 6 mM, about 3 mM to about 5.4 mM, about 4.1 mM to about 50 mM, about 4.1 mM to about 40 mM, about 4.1 mM to about 30 mM, about 4.1 mM to about 20 mM, about 4.1 mM to about 12 mM, about 4.1 mM to about 10 mM, about 4.1 mM to about 7.8 mM, about 4.1 mM to about 6 mM, about 4.1 mM to about 5.4 mM, about 1 mM, about 2.5 mM, about 2.6 mM, about 2.7 mM, about 2.8 mM, about 2.9 mM, about 4.8 mM, about 4.9 mM, about 5 mM, about 5.1 mM, about 5.2 mM, about 5.3 mM, about 5.4 mM, about 7.2 mM, about 7.3 mM, about 7.4 mM, about 7.5 mM, about 7.6 mM, about 7.7 mM, about 7.8 mM, about 9.8 mM, or about 10 mM, based on the total pharmaceutical composition.

The buffer may include one or more (e.g., one, two, or three) selected from the group consisting of phosphate, succinate, and acetate.

In one embodiment, the buffer may include phosphate. In another embodiment, the buffer may include phosphate, and may be free of citrate. In another embodiment, the buffer may include (1) phosphate and (2) succinate and/or acetate, and may be free of citrate.

A concentration of the phosphate may be about 0.1 mM to about 10 mM, about 0.1 mM to about 7.8 mM, about 0.1 mM to about 6 mM, about 0.1 mM to about 4 mM, about 0.1 mM to about 3.3mM, about 0.1 mM to about 3 mM, about 0.1 mM to about 2.7 mM, about 1 mM to about 10 mM, about 1 mM to about 7.8 mM, about 1 mM to about 6 mM, about 1 mM to about 5mM, 1 mM to about 4 mM, about 1 mM to about 3.3mM, about 1 mM to about 3 mM, about 1 mM to about 2.7 mM, about 1.7 mM to about 10 mM, about 1.7 mM to about 7.8 mM, about 1.7 mM to about 6 mM, about 1.7 mM to about 4 mM, about 1.7 mM to about 3.3mM, about 1.7 mM to about 3 mM, about 1.7 mM to about 2.7 mM, about 2 mM to about 10 mM, about 2 mM to about 7.8 mM, about 2 mM to about 6 mM, about 2 mM to about 4 mM, about 2 mM to about 3.3 mM, about 2 mM to about 3 mM, about 2 mM to about 2.7 mM, about 2.5 mM to about 10 mM, about 2.5 mM to about 7.8 mM, about 2.5 mM to about 6 mM, about 2.5 mM to about 2.7 mM, about 1 mM, about 2.5 mM, about 2.6 mM, about 2.7 mM, about 2.8 mM, or about 2.9 mM, based on the total pharmaceutical composition.

A concentration of the succinate may be about 0.1 mM to about 20 mM, about 0.1 mM to about 12 mM, about 0.1 mM to about 10 mM, about 0.1 mM to about 7.8 mM, about 0.1 mM to about 6.2 mM, about 0.1 mM to about 5.4 mM, about 0.1 mM to about 3 mM, about 1 mM to about 20 mM, about 1 mM to about 12 mM, about 1 mM to about 10 mM, about 1 mM to about 7.8 mM, about 1 mM to about 6.2 mM, about 1 mM to about 5.4 mM, about 1 mM to about 3 mM, about 2 mM to about 20 mM, about 2 mM to about 12 mM, about 2 mM to about 10 mM, about 2 mM to about 7.8 mM, about 2 mM to about 6.2 mM, about 2 mM to about 5.4 mM, about 2 mM to about 3 mM, about 2.5 mM to about 20 mM, about 2.5 mM to about 12 mM, about 2.5 mM to about 10 mM, about 2.5 mM to about 7.8 mM, about 2.5 mM to about 6.2 mM, about 2.5 mM to about 5.4 mM, about 2.5 mM to about 3 mM, about 3 mM to about 20 mM, about 3 mM to about 12 mM, about 3 mM to about 10 mM, about 3 mM to about 7.8 mM, about 3 mM to about 6.2 mM, about 3 mM to about 5.4 mM, about 4.1 mM to about 20 mM, about 4.1 mM to about 12 mM, about 4.1 mM to about 10 mM, about 4.1 mM to about 7.8 mM, about 4.1 mM to about 6.2 mM, about 4.1 mM to about 5.4 mM, about 4.8 mM, about 4.9 mM, about 5 mM, about 5.1 mM, about 5.2 mM, about 5.3 mM, or about 5.4 mM, based on the total pharmaceutical composition.

A concentration of the acetate may be about 0.1 mM to about 10 mM, about 0.1 mM to about 7.8 mM, about 0.1 mM to about 6 mM, about 0.1 mM to about 4 mM, about 0.1 mM to about 3.3mM, about 0.1 mM to about 3 mM, about 0.1 mM to about 2.7 mM, about 1 mM to about 10 mM, about 1 mM to about 7.8 mM, about 1 mM to about 6 mM, about 1 mM to about 5mM, 1 mM to about 4 mM, about 1 mM to about 3.3mM, about 1 mM to about 3 mM, about 1 mM to about 2.7 mM, about 1.7 mM to about 10 mM, about 1.7 mM to about 7.8 mM, about 1.7 mM to about 6 mM, about 1.7 mM to about 4 mM, about 1.7 mM to about 3.3mM, about 1.7 mM to about 3 mM, about 1.7 mM to about 2.7 mM, about 2 mM to about 10 mM, about 2 mM to about 7.8 mM, about 2 mM to about 6 mM, about 2 mM to about 4 mM, about 2 mM to about 3.3 mM, about 2 mM to about 3 mM, about 2 mM to about 2.7 mM, about 2.5 mM to about 10 mM, about 2.5 mM to about 7.8 mM, about 2.5 mM to about 6 mM, about 2.5 mM to about 2.7 mM, about 1 mM, about 2.5 mM, about 2.6 mM, about 2.7 mM, about 2.8 mM, or about 2.9 mM, based on the total pharmaceutical composition.

The pharmaceutical composition provided in the present disclosure may be free of citrate, a pharmaceutically acceptable salt thereof, or both thereof as the buffer.

(3) Histidine

In the present disclosure, histidine may be included at a concentration of about 10 mM or more, about 20 mM or more, about 30 mM or more, about 40 mM or more, about 50 mM or more, about 51 mM or more, or about 52 mM or more, e.g., about 10 mM to about 100 mM, about 10 mM to about 90 mM, about 10 mM to about 80 mM, about 10 mM to about 70 mM, about 20 mM to about 100 mM, about 20 mM to about 90 mM, about 20 mM to about 80 mM, about 20 mM to about 70 mM, about 30 mM to about 100 mM, about 30 mM to about 90 mM, about 30 mM to about 80 mM, about 30 mM to about 70 mM, about 40 mM to about 100 mM, about 40 mM to about 90 mM, about 40 mM to about 80 mM, about 40 mM to about 70 mM, about 50 mM to about 100 mM, about 50 mM to about 90 mM, about 50 mM to about 80 mM, about 50 mM to about 70 mM, or about 59 mM, based on the total pharmaceutical composition.

The histidine may be included in the form of histidine and/or in the form of a pharmaceutically acceptable salt (e.g., hydrochloride, etc.) thereof and/or a hydrate (e.g., monohydrate, etc.) thereof (e.g., histidine hydrochloride monohydrate, etc.), but is not limited thereto. Unless specified otherwise, the term "histidine" may be interpreted as including one or more selected from the group consisting of histidine, a pharmaceutically acceptable salt (e.g., hydrochloride, etc.) thereof, and a hydrate (e.g., monohydrate, etc.) thereof (e.g., histidine hydrochloride monohydrate, etc.).

(4) Polyol

In the present disclosure, the polyol may be one or more selected from the group consisting of sugar and sugar alcohols. For example, the polyol may be one or more selected from the group consisting of mannitol, sorbitol, meglumine, trehalose, sucrose, maltose, lactose, glucose, xylitol, arabitol, erythritol, lactitol, maltitol, inositol, etc. In one embodiment, the polyol may be mannitol.

In one embodiment, the polyol may include mannitol, and may be free of one or more selected from sugars and sugar alcohols other than mannitol, for example, one or more selected from the group consisting of sorbitol, trehalose, and sucrose, or all of them.

To more improve the protein stability in the pharmaceutical composition provided in the present disclosure, a concentration of the polyol, for example, mannitol, may be about 1 %(w/v) to about 10 %(w/v), about 1 %(w/v) to about 8 %(w/v), about 1 %(w/v) to about 6 %(w/v), about 1 %(w/v) to about 5 %(w/v), about 1 %(w/v) to about 4.7 %(w/v), about 1 %(w/v) to about 4 %(w/v), about 1 %(w/v) to about 3.5 %(w/v), about 1 %(w/v) to about 3.3 %(w/v), about 1 %(w/v) to about 3 %(w/v), about 2 %(w/v) to about 10 %(w/v), about 2 %(w/v) to about 8 %(w/v), about 2 %(w/v) to about 6 %(w/v), about 2 %(w/v) to about 5 %(w/v), about 2 %(w/v) to about 4.7 %(w/v), about 2 %(w/v) to about 4 %(w/v), about 2 %(w/v) to about 3.5 %(w/v), about 2 %(w/v) to about 3.3 %(w/v), about 2 %(w/v) to about 3 %(w/v), about 2.5 %(w/v) to about 10 %(w/v), about 2.5 %(w/v) to about 8 %(w/v), about 2.5 %(w/v) to about 6 %(w/v), about 2.5 %(w/v) to about 5 %(w/v), about 2.5 %(w/v) to about 4.7 %(w/v), about 2.5 %(w/v) to about 4 %(w/v), about 2.5 %(w/v) to about 3.5 %(w/v), about 2.5 %(w/v) to about 3.3 %(w/v), about 2.5 %(w/v) to about 3 %(w/v), or about 3 %(w/v), based on the total pharmaceutical composition.

(5) Surfactant

In the present disclosure, the surfactant may be selected from all pharmaceutically acceptable surfactants capable of evenly dispersing a protein in a liquid composition medium. The surfactant may be a non-ionic surfactant, and specifically, one or more selected from the group consisting of polysorbates (e.g., polysorbate 20 (polyoxyethylene 20 sorbitan monolaurate), polysorbate 40 (polyethylene 20 sorbitan monopalmitate), polysorbate 60 (polyethylene 20 sorbitan monostearate), polysorbate 65 (polyethylene 20 sorbitan tristearate), polysorbate 80 (polyethylene 20 sorbitan monooleate), polysorbate 85 (polyethylene 20 sorbitan trioleate) (the number (20) following polyoxyethylene refers to the total number of oxyethylene units ($-(CH_2CH_2O)-$)), poloxamer (PEO-PPO-PEO copolymer; PEO: poly(ethylene oxide), PPO: polypropylene oxide)), sorbitan esters (e.g., sorbitan polyethoxylates, etc.), polyethylene-polypropylene glycol, polyoxyethylene compounds (e.g., polyoxyethylene-stearate, polyoxyethylene alkyl ether (alkyl: C1-C30), polyoxyethylene monolauryl ether, alkylphenyl polyoxyethylene copolymer (alkyl: C1-C30), etc.), sodium dodecyl sulphate (SDS), etc. More specifically, to achieve much better formulation stability, the surfactant may be polysorbate 20. In an embodiment, the formulation of the present disclosure may include polysorbate 20, and may be free of polysorbate 80.

To further improve the protein stability in the formulation of the present disclosure, a concentration of the surfactant, for example, polysorbate 20, included in the pharmaceutical composition may be 0.01%(w/v) to 0.2%(w/v), 0.01%(w/v) to 0.1%(w/v), 0.01%(w/v) to 0.08%(w/v), 0.03%(w/v) to 0.2%(w/v), 0.03%(w/v) to 0.1%(w/v), 0.03%(w/v) to 0.08%(w/v), 0.05%(w/v) to 0.2%(w/v), 0.05%(w/v) to 0.1%(w/v), 0.05%(w/v) to 0.08%(w/v), about 0.01%(w/v), about 0.03%(w/v), about 0.05%(w/v), or about 0.08%(w/v), based on the total pharmaceutical composition.

(6) Amino acid

The pharmaceutical composition provided in the present disclosure may be free of one or more (e.g., all) selected from the group consisting of amino acids, except for histidine, and pharmaceutically acceptable salts thereof (for example, the concentration of one or more (e.g., all) selected from the group consisting of amino acids, except for histidine, and pharmaceutically acceptable salts thereof may be about 0 mM). In one embodiment, the pharmaceutical composition provided in the present disclosure may be free of one or more (e.g., all) selected from the group consisting of arginine, lysine, proline, glycine, methionine, glutamine, and pharmaceutically acceptable salts thereof (e.g., hydrochloride salt, hydrates, etc.) (for example, the concentration of one or more (e.g., all) selected from the group consisting of arginine, lysine, proline, glycine, methionine, glutamine, and pharmaceutically acceptable salts thereof (e.g., hydrochloride salt, hydrates, etc.) may be about 0 mM).

(7) Salt

The pharmaceutical composition provided in the present disclosure may be free of any salt other than pharmaceutically acceptable salts of phosphate, succinate, and acetate. The pharmaceutically acceptable salt, which is not included in the pharmaceutical composition provided in the present disclosure, may be one or more selected from the group consisting of sodium chloride, sodium sulfate, and potassium chloride. In one embodiment, the salt, which is not included in the pharmaceutical composition of the present disclosure, may be one or more selected from the group consisting of a pharmaceutically acceptable salt of citrate, a sodium chloride salt, a sodium sulfate salt, and a potassium chloride salt thereof, or all of them.

(8) pH

pH of the pharmaceutical composition provided in the present disclosure may be about 4 to about 8, specifically, about 4.0 to about 7.5, about 4.0 to about 7, about 4.0 to about 6.5, about 4.0 to about 6, about 4.0 to about 5.5, about 4.5 to about 7.5, about 4.5 to about 7, about 4.5 to about 6.5, about 4.5 to about 6, about 4.5 to about 5.5, about 4.8 to about 7.5, about 4.8 to about 7, about 4.8 to about 6.5, about 4.8 to about 6, about 4.8 to about 5.6, about 5.0 to about 7.5, about 5.0 to about 7, about 5.0 to about 6.5, about 5.0 to about 6, about 5.0 to about 5.5, about 5.2 to about 7.5, about 5.2 to about 7.2, about 5.2 to about 7, about 5.2 to about 6.8, about 5.2 to about 6.6, about 5.2 to about 6.5, about 5.5 to about 7.5, about 5.5 to about 7.2, about 5.5 to about 7, about 5.5 to about 6.8, about 5.5 to about 6.6, about 5.5 to about 6.5, about 6.0 to about 7.5, about 6.0 to about 7.2, about 6.0 to about 7, about 6.0 to about 6.8, about 6.0 to about 6.6, about 6.0 to about 6.5, about 6.4 to about 7.5, about 6.4 to about 7.2, about 6.4 to about 7, about 6.4 to about 6.8, about 6.4 to about 6.6, about 6.4 to about 6.5, about 5.2, about 5.3, about 5.4, or about 6.5.

(9) Other additives

The pharmaceutical composition provided in the present disclosure may be free of one or more selected from the group consisting of ammonium salts, for example, ammonium chloride, ammonium sulfate, ammonium carbonate, ammonium nitrate, etc. In another embodiment, the pharmaceutical composition provided in the present disclosure may be free of sodium chloride, sodium sulfate, potassium chloride, sodium hydroxide, potassium hydroxide, ethylenediaminetetraacetic acid (EDTA), or all of them.

(10) Formulation

The pharmaceutical composition provided in the present disclosure may be:

1) a pharmaceutical composition including adalimumab, phosphate, and histidine, wherein pH is 5 to 7;
2) a pharmaceutical composition including adalimumab, phosphate, succinate, and histidine, wherein pH is 5 to 7;
3) a pharmaceutical composition including adalimumab, phosphate, acetate, and histidine, wherein pH is 5 to 7;
4) a pharmaceutical composition including adalimumab, phosphate, histidine, and mannitol, wherein pH is 5 to 7;
5) a pharmaceutical composition including adalimumab, phosphate, succinate, histidine, and mannitol, wherein pH is 5 to 7;
6) a pharmaceutical composition including adalimumab, phosphate, acetate, histidine, and mannitol, wherein pH is 5 to 7;
7) a pharmaceutical composition including adalimumab, phosphate, histidine, and polysorbate 20, wherein pH is 5 to 7;
8) a pharmaceutical composition including adalimumab, phosphate, succinate, histidine, and polysorbate 20, wherein pH is 5 to 7;
9) a pharmaceutical composition including adalimumab, phosphate, acetate, histidine, and polysorbate 20, wherein pH is 5 to 7;
10) a pharmaceutical composition including adalimumab, phosphate, histidine, mannitol, and polysorbate 20, wherein pH is 5 to 7;
11) a pharmaceutical composition including adalimumab, phosphate, succinate, histidine, mannitol, and polysorbate 20, wherein pH is 5 to 7;
12) a pharmaceutical composition including adalimumab, phosphate, acetate, histidine, mannitol, and polysorbate 20, wherein pH is 5 to 7;
13) a pharmaceutical composition including 40 mg/ml to 200 mg/ml of adalimumab, 1.7 mM to 10 mM of phosphate, and 40 mM to 80 mM of histidine, wherein pH is 5 to 7;
14) a pharmaceutical composition including 40 mg/ml to 200 mg/ml of adalimumab, 1.7 mM to 10 mM of phosphate, 4.1 mM to 12 mM of succinate, and 40 mM to 80 mM of histidine, wherein pH is 5 to 7;

15) a pharmaceutical composition including 40 mg/ml to 200 mg/ml of adalimumab, 1.7 mM to 10 mM of phosphate, 1.7 mM to 10 mM of acetate, and 40 mM to 80 mM of histidine, wherein pH is 5 to 7;

16) a pharmaceutical composition including 40 mg/ml to 200 mg/ml of adalimumab, 1.7 mM to 10 mM of phosphate, 40 mM to 80 mM of histidine, and 2 %(w/v) to 5 %(w/v) of mannitol, wherein pH is 5 to 7;

17) a pharmaceutical composition including 40 mg/ml to 200 mg/ml of adalimumab, 1.7 mM to 10 mM of phosphate, 4.1 mM to 12 mM of succinate, 40 mM to 80 mM of histidine, and 2 %(w/v) to 5 %(w/v) of mannitol, wherein pH is 5 to 7;

18) a pharmaceutical composition including 40 mg/ml to 200 mg/ml of adalimumab, 1.7 mM to 10 mM of phosphate, 1.7 mM to 10 mM of acetate, 40 mM to 80 mM of histidine, and 2 %(w/v) to 5 %(w/v) of mannitol, wherein pH is 5 to 7;

19) a pharmaceutical composition including 40 mg/ml to 200 mg/ml of adalimumab, 1.7 mM to 10 mM of phosphate, 40 mM to 80 mM of histidine, and 0.01 %(w/v) to 0.9 %(w/v) of polysorbate 20, wherein pH is 5 to 7;

20) a pharmaceutical composition including 40 mg/ml to 200 mg/ml of adalimumab, 1.7 mM to 10 mM of phosphate, 4.1 mM to 12 mM of succinate, 40 mM to 80 mM of histidine, and 0.01 %(w/v) to 0.9 %(w/v) of polysorbate 20, wherein pH is 5 to 7;

21) a pharmaceutical composition including 40 mg/ml to 200 mg/ml of adalimumab, 1.7 mM to 10 mM of phosphate, 1.7 mM to 10 mM of acetate, 40 mM to 80 mM of histidine, and 0.01 %(w/v) to 0.9 %(w/v) of polysorbate 20, wherein pH is 5 to 7;

22) a pharmaceutical composition including 40 mg/ml to 200 mg/ml of adalimumab, 1.7 mM to 10 mM of phosphate, 40 mM to 80 mM of histidine, 2 %(w/v) to 5 %(w/v) of mannitol, and 0.01 %(w/v) to 0.9 %(w/v) of polysorbate 20, wherein pH is 5 to 7;

23) a pharmaceutical composition including 40 mg/ml to 200 mg/ml of adalimumab, 1.7 mM to 10 mM of phosphate, 4.1 mM to 12 mM of succinate, 40 mM to 80 mM of histidine, 2 %(w/v) to 5 %(w/v) of mannitol, and 0.01 %(w/v) to 0.9 %(w/v) of polysorbate 20, wherein pH is 5 to 7;

24) a pharmaceutical composition including 40 mg/ml to 200 mg/ml of adalimumab, 1.7 mM to 10 mM of phosphate, 1.7 mM to 10 mM of acetate, 40 mM to 80 mM of histidine, 2 %(w/v) to 5 %(w/v) of mannitol, and 0.01 %(w/v) to 0.9 %(w/v) of polysorbate 20, wherein pH is 5 to 7;

25) a pharmaceutical composition including 100 mg/ml of adalimumab, 2.6 mM of phosphate, and 59 mM of histidine, wherein pH is 5 to 7;

26) a pharmaceutical composition including 100 mg/ml of adalimumab, 2.6 mM of phosphate, 5 mM of succinate, and 59 mM of histidine, wherein pH is 5 to 7;

27) a pharmaceutical composition including 100 mg/ml of adalimumab, 2.6 mM of phosphate, 2.8 mM of acetate, and 59 mM of histidine, wherein pH is 5 to 7;

28) a pharmaceutical composition including 100 mg/ml of adalimumab, 2.6 mM of phosphate, 59 mM of histidine, and 3 %(w/v) of mannitol, wherein pH is 5 to 7;

29) a pharmaceutical composition including 100 mg/ml of adalimumab, 2.6 mM of phosphate, 5 mM of succinate, 59 mM of histidine, and 3 %(w/v) of mannitol, wherein pH is 5 to 7;

30) a pharmaceutical composition including 100 mg/ml of adalimumab, 2.6 mM of phosphate, 2.8 mM of acetate, 59 mM of histidine, and 3 %(w/v) of mannitol, wherein pH is 5 to 7;

31) a pharmaceutical composition including 100 mg/ml of adalimumab, 2.6 mM of phosphate, 59 mM of histidine, and 0.08 %(w/v) of polysorbate 20, wherein pH is 5 to 7;

32) a pharmaceutical composition including 100 mg/ml of adalimumab, 2.6 mM of phosphate, 5 mM of succinate, 59 mM of histidine, and 0.08 % (w/v) of polysorbate 20, wherein pH is 5 to 7;

33) a pharmaceutical composition including 100 mg/ml of adalimumab, 2.6 mM of phosphate, 2.8 mM of acetate, 59 mM of histidine, and 0.08 %(w/v) of polysorbate 20, wherein pH is 5 to 7;

34) a pharmaceutical composition including 100 mg/ml of adalimumab, 2.6 mM of phosphate, 59 mM of histidine, 3 %(w/v) of mannitol, and 0.08 %(w/v) of polysorbate 20, wherein pH is 5 to 7;

35) a pharmaceutical composition including 100 mg/ml of adalimumab, 2.6 mM of phosphate, 5 mM of succinate, 59 mM of histidine, 3 %(w/v) of mannitol, and 0.08 %(w/v) of polysorbate 20, wherein pH is 5 to 7;

36) a pharmaceutical composition including 100 mg/ml of adalimumab, 2.6 mM of phosphate, 2.8 mM of acetate, 59 mM of histidine, 2 %(w/v) to 5 %(w/v) of mannitol, and 0.08 %(w/v) of polysorbate 20, wherein pH is 5 to 7;

37) a pharmaceutical composition including 50 mg/ml of adalimumab, 2.6 mM of phosphate, and 59 mM of histidine, wherein pH is 5 to 7;

38) a pharmaceutical composition including 50 mg/ml of adalimumab, 2.6 mM of phosphate, 5 mM of succinate, and 59 mM of histidine, wherein pH is 5 to 7;

39) a pharmaceutical composition including 50 mg/ml of adalimumab, 2.6 mM of phosphate, 2.8 mM of acetate, and 59 mM of histidine, wherein pH is 5 to 7;

40) a pharmaceutical composition including 50 mg/ml of adalimumab, 2.6 mM of phosphate, 59 mM of histidine, and 3 %(w/v) of mannitol, wherein pH is 5 to 7;

41) a pharmaceutical composition including 50 mg/ml of adalimumab, 2.6 mM of phosphate, 5 mM of succinate, 59 mM of histidine, and 3 %(w/v) of mannitol, wherein pH is 5 to 7;

42) a pharmaceutical composition including 50 mg/ml of adalimumab, 2.6 mM of phosphate, 2.8 mM of acetate, 59 mM of histidine, and 3 %(w/v) of mannitol, wherein pH is 5 to 7;

43) a pharmaceutical composition including 50 mg/ml of adalimumab, 2.6 mM of phosphate, 59 mM of histidine, and 0.08 %(w/v) of polysorbate 20, wherein pH is 5 to 7;

44) a pharmaceutical composition including 50 mg/ml of adalimumab, 2.6 mM of phosphate, 5 mM of succinate, 59 mM of histidine, and 0.08 %(w/v) of polysorbate 20, wherein pH is 5 to 7;

45) a pharmaceutical composition including 50 mg/ml of adalimumab, 2.6 mM of phosphate, 2.8 mM of acetate, 59 mM of histidine, and 0.08 %(w/v) of polysorbate 20, wherein pH is 5 to 7;

46) a pharmaceutical composition including 50 mg/ml of adalimumab, 2.6 mM of phosphate, 59 mM of histidine, 3 %(w/v) of mannitol, and 0.08 %(w/v) of polysorbate 20, wherein pH is 5 to 7;

47) a pharmaceutical composition including 50 mg/ml of adalimumab, 2.6 mM of phosphate, 5 mM of succinate, 59 mM of histidine, 3 %(w/v) of mannitol, and 0.08 %(w/v) of polysorbate 20, wherein pH is 5 to 7; or

48) a pharmaceutical composition including 50 mg/ml of adalimumab, 2.6 mM of phosphate, 2.8 mM of acetate, 59 mM of histidine, 2 %(w/v) to 5 %(w/v) of mannitol, and 0.08 %(w/v) of polysorbate 20, wherein pH is 5 to 7.

.

In addition, the pharmaceutical compositions of 1) to 48) provided in the present disclosure may be free of:

i) salts other than pharmaceutically acceptable salts of phosphate, succinate, and acetate, and/or

ii) amino acids other than histidine.

Meanwhile, the salts other than pharmaceutically acceptable salts of phosphate, succinate, and acetate may be one or more selected from the group consisting of sodium chloride, sodium sulfate, and potassium chloride.

(11) Stability

The pharmaceutical composition provided in the present disclosure has excellent stability. Even when the pharmaceutical composition includes a relatively high concentration of a protein (specifically, 40 mg/ml or more, 50 mg/ml or more, 75 mg/ml or more, 100 mg/ml or more, or 150 mg/ml or more), it exhibits excellent stability.

As used herein, "excellent stability" or "stably maintained" may mean that a structure, and/or physical, chemical, and/or biological properties of the protein in the composition are maintained during storage (for example, low protein polymer formation rate, low protein aggregation rate, low protein degradation rate, high protein (drug) contents, low denaturation rates, low oxidation rates of amino acids (e.g., methionine residue), etc., during storage).

As used herein, the term 'aggregate' may refer to a polymer product (high molecular weight; HMW) resulting from aggregation of the anti-TNFa antibody proteins which are included in the pharmaceutical composition provided in the present disclosure. In the present disclosure, a protein aggregation rate may be represented as a content of an aggregate (% HMW or HMW%) in the composition at a given time point. A variation in the protein aggregation rate may be represented as a change or difference between the aggregate (polymer product) content (%HMW or HMW%) in the composition at an initial time of storage and the aggregate content (%HMW or HMW%) in the composition after a predetermined period of storage (Δ%HMW or ΔHMW % = [aggregate content (%HMW or HMW%) in the composition at the initial time of storage]-[aggregate content (%HMW or HMW %) in the composition after a predetermined period of storage]).

As used herein, the term 'degradation product' refers to a low-molecular weight product resulting from the degradation of the anti-TNFa antibody protein included in the pharmaceutical composition provided in the present disclosure. In the present disclosure, the protein degradation rate may be represented as a content of the degradation product (%LMW or LMW%) in the composition at a given time point. A variation in the protein degradation rate may be represented as a change or difference between the degradation product (low-molecular-weight polymer product) content (%LMW or LMW%) in the composition at an initial time of storage and the degradation product content (%LMW or LMW%) in the composition after a predetermined period of storage (Δ%LMW or ΔLMW% = [degradation product content (%LMW or LMW%) in composition at the initial time of storage]-[degradation product content (%LMW or LMW%) in composition after a predetermined time period of storage]).

As used herein, the term 'amino acid oxidation rate' refers to a ratio of oxidized amino acids (e.g., a ratio of oxidized methionine) in the amino acids of the anti-TNFa antibody protein included in the pharmaceutical composition provided in the present disclosure, and the term 'variation in the amino acid oxidation rate' refers to a change in the ratio. Such oxidation of amino acids (e.g., methionine) may take place under photostress conditions, such as light exposure. In one embodiment, the amino acid oxidation rate means an oxidation ratio of methionine ($Met_{256}$) which is the $256^{th}$ amino acid residue of adalimumab which is the anti-TNFa antibody (%Met256; a ratio of oxidized methionine (Met256)), and a variation in the amino acid oxidation rate refers to a change in the oxidation ratio of Met256 ($\Delta$%$Met_{256}$). When the amino acid oxidation is attributed to photostress (light exposure), a variation in the amino acid oxidation rate (e.g., $\Delta$%$Met_{256}$) may be represented as a difference ([Light-exposed %Met256] - [Base %Met256], or [Light-exposed %Met256] - [Dark-control %Met256]) in the amino acid oxidation rate (%Met256) between a light exposure group under photostress conditions and a base group (stored at $5\pm3$ °C without light exposure) and/or a dark control (wrapped with foil and exposed to light under the same conditions as the light exposure group ($25\pm2$ °C / $60\pm5$% RH)).

In one embodiment, the pharmaceutical composition provided in the present disclosure may have an adalimumab aggregation rate of about 15 % or less, about 14 % or less, about 13 % or less, about 12 % or less, about 11 % or less, about 10 % or less, about 9 % or less, or about 9 % or less under photostress conditions described in Table 1 of Reference Example 2 below.

Further, in one embodiment, the pharmaceutical composition provided in the present disclosure may have a variation in the adalimumab aggregation rate ($\Delta$%HMW: [Light-exposed %HMW] - [Base %HMW], or [Light-exposed %HMW] - [Dark-control %HMW]) of about 17% or less, about 16% or less, about 15% or less, about 14% or less, about 13% or less, about 12% or less, about 10% or less, about 9.5 or less, about 9% or less, or about 8.5% or less, for example, about 1% to about 17%, about 1% to about 16%, about 1% to about 15%, about 1% to about 14%, about 1% to about 13%, about 1% to about 12%, about 1% to about 10%, about 1% to about 9.5, about 1% to about 9%, about 1% to about 8.5% or less, about 3% to about 17%, about 3% to about 16%, about 3% to about 15%, about 3% to about 14%, about 3% to about 13%, about 3% to about 12%, about 3% to about 10%, about 3% to about 9.5, about 3% to about 9%, or about 3% to about 8.5% under photostress conditions described in Table 1 of Reference Example 2 below.

In another embodiment, the pharmaceutical composition provided in the present disclosure may have a variation in the adalimumab degradation rate ($\Delta$%LMW: [Light-exposed %LMW] - [Base %LMW], or [Light-exposed %LMW] - [Dark-control %LMW]) of about 1.6 % or less, about 1.5 % or less, about 1.4 % or less, or about 1.3 % or less, for example, about 0.1% to about 1.6%, about 0.1% to about 1.5%, about 0.1% to about 1.4%, about 0.1% to about 1.3%, about 0.5% to about 1.6%, about 0.5% to about 1.5%, about 0.5% to about 1.4%, or about 0.5% to about 1.3% under photostress conditions described in Table 1 of Reference Example 2 below.

In another embodiment, the pharmaceutical composition may have a variation in the amino acid oxidation rate ($\Delta$%$Met_{256}$: [Light-exposed %Met256] - [Base %Met256], or [Light-exposed %Met256] - [Dark-control %Met256]) of about 68% or less, about 67% or less, about 66% or less, about 65% or less, about 63% or less, about 60% or less, about 58% or less, about 55% or less, about 54% or less, or about 53% or less, for example, about 10% to about 68%, about 10% to about 67%, about 10% to about 66%, about 10% to about 65%, about 10% to about 63%, about 10% to about 60%, about 10% to about 58%, about 10% to about 55%, about 10% to about 54%, about 10% to about 53%, about 20% to about 68%, about 20% to about 67%, about 20% to about 66%, about 20% to about 65%, about 20% to about 63%, about 20% to about 60%, about 20% to about 58%, about 20% to about 55%, about 20% to about 54%, about 20% to about 53%, about 30% to about 68%, about 30% to about 67%, about 30% to about 66%, about 30% to about 65%, about 30% to about 63%, about 30% to about 60%, about 30% to about 58%, about 30% to about 55%, about 30% to about 54%, about 30% to about 53%, about 40% to about 68%, about 40% to about 67%, about 40% to about 66%, about 40% to about 65%, about 40% to about 63%, about 40% to about 60%, about 40% to about 58%, about 40% to about 55%, about 40% to about 54%, or about 40% to about 53% under photostress conditions described in Table 1 of Reference Example 2 below.

(12) Device

An embodiment provides a device, a kit, or a container, each including the pharmaceutical composition provided in the present disclosure. For example, the device may include the pharmaceutical composition in a container selected from the group consisting of a syringe, a pre-filled syringe, an auto-injector, a bottle, a vial, and a tube.

The device may be mainly used for parenteral administration (for example, subcutaneous, intramuscular, intravenous, intraperitoneal, intra-cerebrospinal, intraarticular, intrasynovial, and/or intrameningeal administration), and may be in the form of a vial, a pack syringe (e.g., pre-filled syringe; needle size: 20G to 40G, e.g., 25G, 26G, 27G, 28G, 29G, or 30G), etc., or may include one or more unit dosage forms including the anti-TNFa antibody (e.g., adalimumab). The unit dosage form may include a ready-to-inject-syringe including a syringe containing a unit dose (single dose) of the pharmaceutical composition. The device may be accompanied with an instruction (guide) for administration.

(13) Treatment of disease

An embodiment provides a pharmaceutical composition for treating a disease, the pharmaceutical composition including the above-described pharmaceutical composition or the device including the same. Another embodiment provides a method of treating a disease, the method including administering a pharmaceutically effective amount of the pharmaceutical composition to a subject in need of administration of the anti-TNFa antibody, e.g., adalimumab. The treatment method may further include identifying the subject in need of administration of the anti-TNFa antibody, e.g., adalimumab, prior to the administering.

The disease may be selected from diseases on which a therapeutic effect may be obtained by administering the anti-TNFa antibody, e.g., adalimumab. The disease may be an immune-related disease, for example, may be selected from the group consisting of arthritis (e.g., rheumatoid arthritis, psoriatic arthritis, idiopathic arthritis, juvenile idiopathic arthritis, enthesitis-related arthritis, etc.), axial spondyloarthritis (e.g., ankylosing spondylitis, severe axial spondyloarthritis other than ankylosing spondylitis, etc.), Crohn's disease (e.g., adult (18 years or older) Crohn's disease, juvenile (6-17 years old) Crohn's disease), psoriasis (e.g., adult (18 years or older), or juvenile (4-17 years old) plaque psoriasis), hidradenitis suppurativa, ulcerative colitis, etc.

The subject who needs the administration of the anti-TNFa antibody, e.g., adalimumab, may be selected from mammals including humans suffering from a disease or a disorder that may be significantly treated (removal, reduction, relief, or alleviation of symptoms) by administering the anti-TNFa antibody, e.g., adalimumab. The disease or disorder may be selected from the above-described diseases.

(14) Administration Route, Administration Dose, and Formulation

The pharmaceutical composition provided in the present disclosure may be administered via a parenteral route. Parenteral administration (for example, injection) may be performed via an intravenous route or a subcutaneous route. Parenteral administration may be a bolus injection or a continuous injection.

The pharmaceutical composition provided in the present disclosure may be formulated into a form suitable for the above administration route. In one embodiment, the pharmaceutical composition may be formulated into an injection agent, or an injectable ready-to-use form, but is not limited thereto.

In another embodiment, the pharmaceutical composition may be formulated such that the entire amount or a pharmaceutically effective amount of the anti-TNFa antibody, e.g., adalimumab, may be included in a single dosage form or allocated into two or more (e.g., 2, 3, 4, 5, 6, 7, 8, 9, or 10) dosage forms. The formulated composition may be included in the above-described device as a unit dosage form.

In still another embodiment, the pharmaceutical composition may be formulated into a dosage form that allows the anti-TNFa antibody, e.g., adalimumab included in therein to be administered into a body all at once (e.g., within 1 minute, 30 seconds, 20 seconds, or 10 seconds) or slowly over 5 minutes or longer, 10 minutes or longer, 30 minutes or longer, 60 minutes or longer, 90 minutes or longer, 120 minutes or longer, 150 minutes or longer, 180 minutes or longer, 210 minutes or longer, or 240 m minutes or longer, but is not limited thereto.

The subject to whom the pharmaceutical composition is administered may be selected from mammals including primates (humans, etc.), rodents (mice, rats, guinea pigs, hamsters, rabbits, etc.), cats, dogs, pigs, cow, horses, etc.

The pharmaceutically effective amount of the pharmaceutical composition provided in the present disclosure or the anti-TNFa antibody (e.g., adalimumab) included in the pharmaceutical composition provided in the present disclosure may refer to an amount or a dose that may exhibit a desired pharmacological effect, for example, removal, reduction, relief, or alleviation of symptoms. The pharmaceutically effective amount may be determined depending on various factors including a formulation method, an administration manner, a patient's age, body weight, gender, illness state (severity of symptom), diets, administration time, administration interval, administration routes, excretion rate, responsiveness, previous therapy, clinical history, etc. The doses may be determined according to the prescription of the attending physician. The pharmaceutically effective amount may be administered once or in a series of administrations of twice or more by allocating.

In one embodiment, the pharmaceutical composition may be administered once to three times per a week over three weeks or more at such a dose as to include about 200 mg or less, about 150 mg or less, about 100 mg, about 50 mg, or 40 mg of the anti-TNFa antibody (e.g., adalimumab). In another embodiment, the pharmaceutical composition may be prepared in common bulk formulations. The concentrations of the components of the pharmaceutical composition may be adjusted to a level higher than that required for administration, and properly diluted prior to administration.

ADVANTAGEOUS EFFECTS OF DISCLOSURE

The present disclosure provides an aqueous liquid composition, specifically, a pharmaceutical composition capable of stably maintaining physical, chemical, and/or biological activities of a protein, e.g., an anti-TNFa antibody. The composition may prevent oxidation of amino acid residues, which may occur during the storage of proteins, and may inhibit formation of polymers and/or aggregates, formation of degradation products (fragments), etc., thereby stably maintaining the pharmaceutical efficacy of the protein.

BRIEF DESCRIPTION OF DRAWINGS

FIG. 1A is a graph showing the high molecular weight% (%HMW) of a protein in an antibody formulation according to one embodiment under heat stress conditions (storage at 40 °C for 4 weeks) depending on the type of buffer and the presence/absence and concentration of histidine;

FIG. 1B is a graph showing the monomer weight% (%Monomer) of a protein in an antibody formulation according to one embodiment under heat stress conditions (storage at 40 °C for 4 weeks) depending on the type of buffer and the presence/absence and concentration of histidine;

FIG. 1C is a graph showing low molecular weight% (%LMW) of a protein in an antibody formulation according to one embodiment under heat stress conditions (storage at 40 °C for 4 weeks) depending on the type of buffer and the presence/absence and concentration of histidine;

FIG. 2A is a graph showing %HMW of a protein in an antibody formulation according to one embodiment under freeze/thaw conditions depending on the type of buffer and the presence/absence and concentration of histidine;

FIG. 2B is a graph showing %Monomer of a protein in an antibody formulation according to one embodiment under freeze/thaw conditions depending on the type of buffer and the presence/absence and concentration of histidine;

FIG. 2C is a graph showing %LMW of a protein in an antibody formulation according to one embodiment under freeze/thaw conditions depending on the type of buffer and the presence/absence and concentration of histidine;

FIG. 3A is a graph showing %HMW of a protein in an antibody formulation according to one embodiment under photostress conditions depending on the presence/absence and concentration of histidine;

FIG. 3B is a graph showing %Monomer of a protein in an antibody formulation according to one embodiment under photostress conditions depending on the presence/absence and concentration of histidine;

FIG. 3C is a graph showing %LMW of a protein in an antibody formulation according to one embodiment under photostress conditions depending on the presence/absence and concentration of histidine;

FIG. 4 is a graph showing an oxidation rate (%Met$_{256/\text{light-exposured}}$) of an amino acid (Met$_{256}$) of an antibody formulation according to one embodiment under photostress conditions depending on the presence/absence and concentration of histidine;

FIG. 5A is a graph showing %HMW of a protein in an antibody formulation according to one embodiment under heat stress conditions (storage at 40 °C for 4 weeks) depending on the type of polyol;

FIG. 5B is a graph showing %Monomer of a protein in an antibody formulation according to one embodiment under heat stress conditions (storage at 40 °C for 4 weeks) depending on the type of polyol; and

FIG. 5C is a graph showing %LMW of a protein in an antibody formulation according to one embodiment under heat stress conditions (storage at 40 °C for 4 weeks) depending on the type of polyol.

DETAILED DESCRIPTION

Hereinafter, the present disclosure will be described in more detail with reference to Examples and Experimental Examples. These Examples and Experimental Examples are only for illustrating the present disclosure, and they are not to be construed as limiting the present disclosure.

REFERENCE EXAMPLES

Reference Example 1. SE-HPLC Analysis

Size exclusion-high performance liquid chromatography (SE-HPLC) analysis was performed using the HPLC system of Waters Corporation according to the manufacturer's manual. A total of three peaks were separated depending on retention times (molecular weights of proteins). These three peaks accounted for a HMW peak (protein aggregation), a monomer peak, and an LMW peak (protein degradation), in order of short retention times (large molecular weights of proteins).

- %HMW (High molecular weight%) = {area of HMW/area of (HMW + monomer + LMW)}*100)
- %Monomer (Monomer Weight%) = {area of monomer/area of (HMW + monomer + LMW)}*100)
- %LMW(Low molecular weight%) = {area of LMW/area of (HMW + monomer + LMW)}*100)

Reference Example 2: Photostress

Formulations were treated with photostress by being exposed to light under conditions of Table 1 below (light-exposed group):

[Table 1]

| Description | Actual Expose |
|---|---|
| Cool White Fluorescent Lamp | 1.6 M lux hours |
| Near Ultraviolet Lamp | 260 Watt hours/square meter |
| Chamber Environmental Conditions | 25±2°C/60±5% RH |

Conditions for controls except the light-exposure group are as follows:

Base: stored at 5±3 °C without light exposure;
Dark control: wrapped up with foil and exposed to light under the same conditions as the light-exposed group (25±2 °C / 60±5% RH)

Reference Example 3: Measurement of %Oxidation Rate (%Oxidation Level) of Amino Acid Residue

With regard to %oxidation rate, the mass of the 256th methionine amino acid residue of adalimumab was measured using liquid-chromatography-mass spectrometry (LC-MS) of Waters Corporation. After performing the LC-MS experiment, relative %oxidation rate was determined using MassLynx™ program.

Relative %Oxidation = (Control intensity of oxidized peptide*100)/{(Control intensity of non-oxidized peptide)+(Control intensity of oxidized peptide)

Example 1. Buffer and Amino Acid Screening

1.1. Preparation of Formulation

To reduce pain upon antibody administration, efforts were made to reduce the content of citrate in formulations. In this regard, appropriate formulation ingredients were selected to develop a citrate-free formulation.

In this exemplary embodiment, appropriate buffers and amino acids were selected. To this end, aqueous liquid formulations having compositions of the following Table 2 were prepared using an anti-TNFa antibody (Adalimumab; anti-TNF$\alpha$ monoclonal antibody; CAS Registry Number: 331731-18-1) as a protein:

[Table 2]

| Sample No. | Protein Conc. (mg/mL) | pH | Buffer | Excipient 1 | Excipient 2 | Surfactant |
|---|---|---|---|---|---|---|
| 1 | 100 | 5.2 | 2.6 mM Na-phosphate | 59 mM His | 3.0% mannitol | 0.08% PS 20 |
| 2 | | | 2.6 mM Na-phosphate + 5 mM Na-succinate | | | |
| 3 | | | | N/A | 5.0% mannitol | |

(continued)

| Sample No. | Protein Conc. (mg/mL) | pH | Buffer | Excipient 1 | Excipient 2 | Surfactant |
|---|---|---|---|---|---|---|
|  |  |  |  |  |  |  |
| 4 |  |  |  | 59 mM Arg-HCl | 3.0% mannitol |  |
| 5 |  |  |  | 105 mM Met |  |  |
| 6 |  |  |  | 10 mM His | 4.5% mannitol |  |
| 7 |  |  |  | 80 mM His | 2.0% mannitol |  |
| 8 |  |  | 2.6 mM Na-phosphate, 2.8 mM Na-acetate | 59 mM His | 3.0% mannitol |  |
| 9 |  |  | 10 mM acetic acid, NaOH | N/A | 9.0% sucrose | 0.1% PS80 |
| 10 |  |  | 24.7 mM Na-acetate | N/A | 8.1% trehalose |  |
|  |  |  |  |  |  |  |
| 11* |  |  | N/A | N/A | 4.2% mannitol |  |
| [000188] (*: a sample including the same formulation ingredients as Humira® including 100 mg/mL of adalimumab; [000189] N/A: not applicable; [000190] %: %(w/v), hereinafter, % accounts for %(w/v) when it is used for indicating contents of formulation ingredients) |||||||

1.2. Stability under Heat Stress Conditions

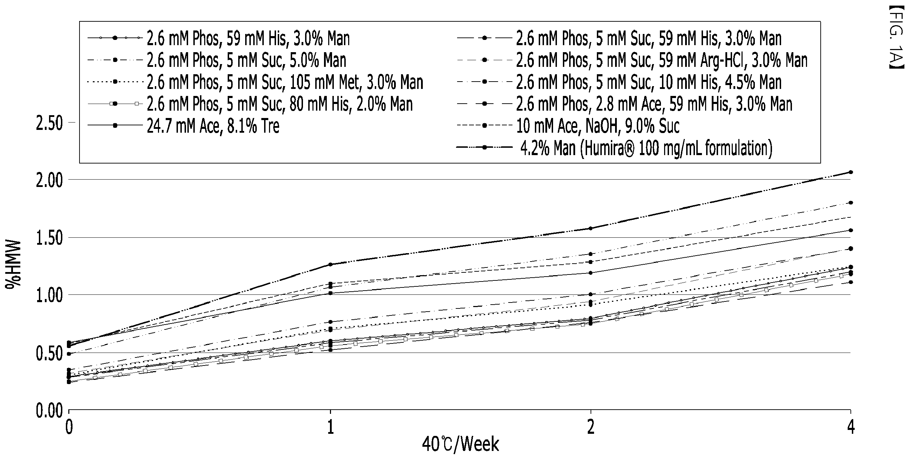

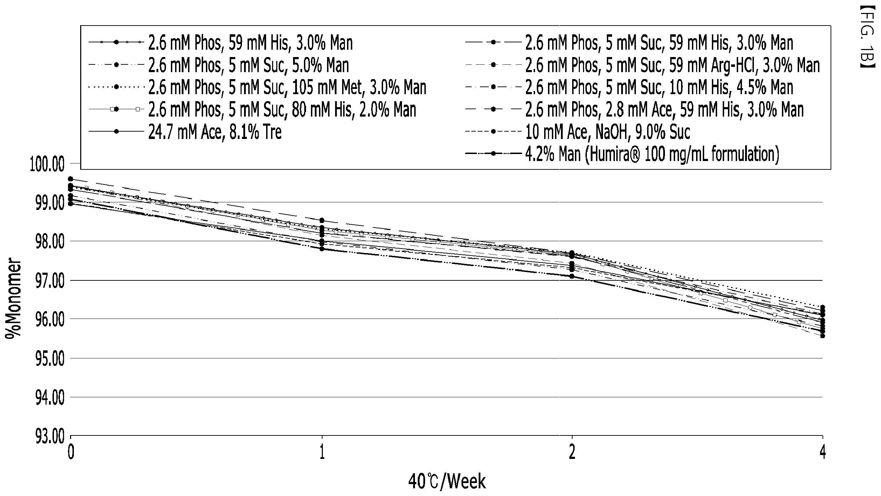

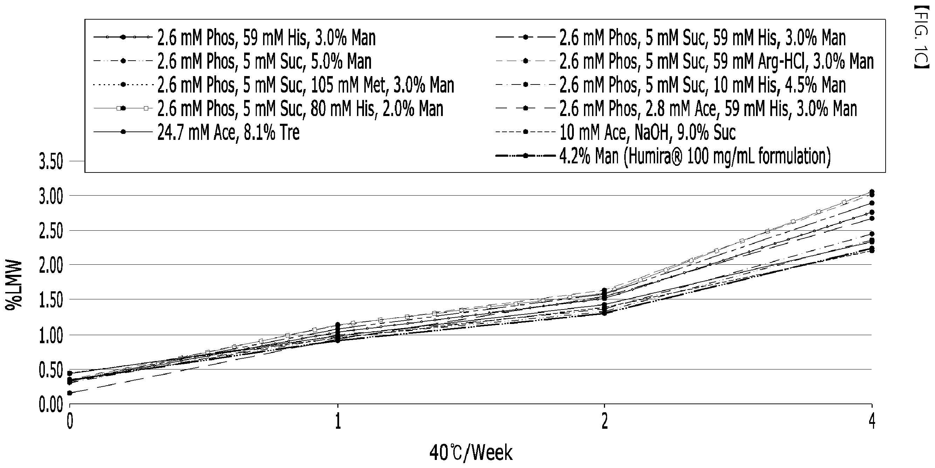

The formulations prepared in Example 1.1 were stored at 40 °C for four weeks, and then high molecular weight% (%HMW), monomer weight% (%Monomer), and low molecular weight% (%LMW) of the protein included in each formulation were measured by SE-HPLC analysis according to Reference Example 1. In addition, variations over the four weeks (represented as Δ%HMW, Δ%Monomer, and Δ%LMW, respectively) were measured. Δ%HMW, Δ%Monomer, and Δ%LMW were obtained as differences resulting from subtracting %HMW, %Monomer, and %LMW at 0(initial) week from %HMW, %Monomer, and %LMW at $4^{th}$ week, respectively. The experiment was repeated three times (n=3) and the average values of the obtained results are shown in the following Tables 3 to 5, and FIGS. 1A to 1C.

[Table 3]

| %HMW upon Storage over Four Weeks at 40 °C | | | | | |
|---|---|---|---|---|---|
| Sample No. | %HMW | | | | Δ%HMW |
|  | 0(initial) | 1 wk | 2 wk | 4 wk |  |
| 1 | 0.29 | 0.60 | 0.79 | 1.24 | 0.95 |
| SD | 0.01 | 0.03 | 0.06 | 0.04 | 0.03 |
| 2 | 0.28 | 0.59 | 0.78 | 1.20 | 0.91 |
| SD | 0.02 | 0.01 | 0.02 | 0.03 | 0.03 |
| 3 | 0.49 | 1.06 | 1.36 | 1.81 | 1.32 |
| SD | 0.01 | 0.04 | 0.02 | 0.04 | 0.03 |
| 4 | 0.32 | 0.70 | 0.94 | 1.41 | 1.09 |

(continued)

| %HMW upon Storage over Four Weeks at 40 °C | | | | | |
|---|---|---|---|---|---|
| Sample No. | %HMW | | | | Δ%HMW |
| | 0(initial) | 1 wk | 2 wk | 4 wk | |
| SD | 0.00 | 0.01 | 0.02 | 0.04 | 0.04 |
| 5 | 0.31 | 0.71 | 0.92 | 1.25 | 0.94 |
| SD | 0.01 | 0.01 | 0.02 | 0.01 | 0.00 |
| 6 | 0.35 | 0.77 | 1.00 | 1.40 | 1.05 |
| SD | 0.01 | 0.01 | 0.02 | 0.02 | 0.02 |
| 7 | 0.25 | 0.56 | 0.75 | 1.18 | 0.93 |
| SD | 0.02 | 0.03 | 0.04 | 0.03 | 0.05 |
| 8 | 0.24 | 0.52 | 0.76 | 1.11 | 0.88 |
| SD | 0.01 | 0.01 | 0.00 | 0.01 | 0.01 |
| 9 | 0.58 | 1.10 | 1.28 | 1.68 | 1.10 |
| SD | 0.02 | 0.01 | 0.01 | 0.04 | 0.05 |
| 10 | 0.59 | 1.01 | 1.19 | 1.56 | 0.98 |
| SD | 0.05 | 0.05 | 0.03 | 0.06 | 0.01 |
| 11 | 0.56 | 1.27 | 1.58 | 2.07 | 1.51 |
| SD | 0.01 | 0.02 | 0.01 | 0.04 | 0.04 |

[Table 4]

| %Monomer upon Storage over Four Weeks at 40 °C | | | | | |
|---|---|---|---|---|---|
| Sample No. | %Monomer | | | | Δ%Monomer |
| | 0(initial) | 1 wk | 2 wk | 4 wk | |
| 1 | 99.39 | 98.36 | 97.68 | 95.99 | -3.39 |
| SD | 0.03 | 0.03 | 0.10 | 0.05 | 0.07 |
| 2 | 99.38 | 98.32 | 97.62 | 95.90 | -3.48 |
| SD | 0.03 | 0.02 | 0.03 | 0.03 | 0.01 |
| 3 | 99.18 | 97.98 | 97.28 | 95.83 | -3.35 |
| SD | 0.02 | 0.04 | 0.04 | 0.05 | 0.05 |
| 4 | 99.33 | 98.16 | 97.43 | 95.59 | -3.74 |
| SD | 0.01 | 0.01 | 0.06 | 0.10 | 0.10 |
| 5 | 99.35 | 98.33 | 97.71 | 96.30 | -3.05 |
| SD | 0.01 | 0.01 | 0.03 | 0.01 | 0.01 |
| 6 | 99.33 | 98.23 | 97.62 | 96.14 | -3.19 |
| SD | 0.03 | 0.01 | 0.01 | 0.02 | 0.05 |
| 7 | 99.42 | 98.29 | 97.65 | 95.76 | -3.66 |
| SD | 0.04 | 0.05 | 0.05 | 0.03 | 0.07 |
| 8 | 99.60 | 98.54 | 97.70 | 96.22 | -3.38 |

(continued)

| %Monomer upon Storage over Four Weeks at 40 °C | | | | | |
|---|---|---|---|---|---|
| Sample No. | %Monomer | | | | Δ%Monomer |
| | 0(initial) | 1 wk | 2 wk | 4 wk | |
| SD | 0.01 | 0.01 | 0.01 | 0.02 | 0.03 |
| 9 | 98.97 | 97.93 | 97.32 | 96.11 | -2.86 |
| SD | 0.04 | 0.03 | 0.01 | 0.06 | 0.10 |
| 10 | 98.96 | 98.00 | 97.37 | 96.10 | -2.86 |
| SD | 0.05 | 0.03 | 0.01 | 0.04 | 0.08 |
| 11 | 99.09 | 97.82 | 97.11 | 95.68 | -3.40 |
| SD | 0.02 | 0.02 | 0.02 | 0.04 | 0.03 |

[Table 5]

| %LMW upon Storage over Four Weeks at 40 °C | | | | | |
|---|---|---|---|---|---|
| Sample No. | %LMW | | | | Δ%LMW |
| | 0(initial) | 1 wk | 2 wk | 4 wk | |
| 1 | 0.33 | 1.04 | 1.53 | 2.76 | 2.44 |
| SD | 0.02 | 0.03 | 0.04 | 0.02 | 0.04 |
| 2 | 0.33 | 1.09 | 1.60 | 2.90 | 2.56 |
| SD | 0.03 | 0.01 | 0.03 | 0.02 | 0.03 |
| 3 | 0.34 | 0.96 | 1.35 | 2.36 | 2.02 |
| SD | 0.01 | 0.01 | 0.03 | 0.01 | 0.00 |
| 4 | 0.35 | 1.14 | 1.64 | 3.01 | 2.67 |
| SD | 0.01 | 0.01 | 0.05 | 0.07 | 0.08 |
| 5 | 0.34 | 0.96 | 1.37 | 2.45 | 2.11 |
| SD | 0.00 | 0.01 | 0.02 | 0.01 | 0.01 |
| 6 | 0.32 | 1.00 | 1.37 | 2.45 | 2.13 |
| SD | 0.02 | 0.01 | 0.01 | 0.03 | 0.05 |
| 7 | 0.33 | 1.15 | 1.60 | 3.06 | 2.73 |
| SD | 0.02 | 0.02 | 0.05 | 0.01 | 0.03 |
| 8 | 0.16 | 0.94 | 1.54 | 2.67 | 2.51 |
| SD | 0.01 | 0.01 | 0.01 | 0.02 | 0.02 |
| 9 | 0.45 | 0.96 | 1.40 | 2.22 | 1.77 |
| SD | 0.03 | 0.01 | 0.02 | 0.03 | 0.05 |
| 10 | 0.45 | 0.99 | 1.44 | 2.33 | 1.89 |
| SD | 0.01 | 0.03 | 0.05 | 0.09 | 0.09 |
| 11 | 0.35 | 0.92 | 1.31 | 2.25 | 1.89 |
| SD | 0.03 | 0.00 | 0.02 | 0.02 | 0.04 |

1.3. Stability of Formulation under Freeze/Thaw Conditions

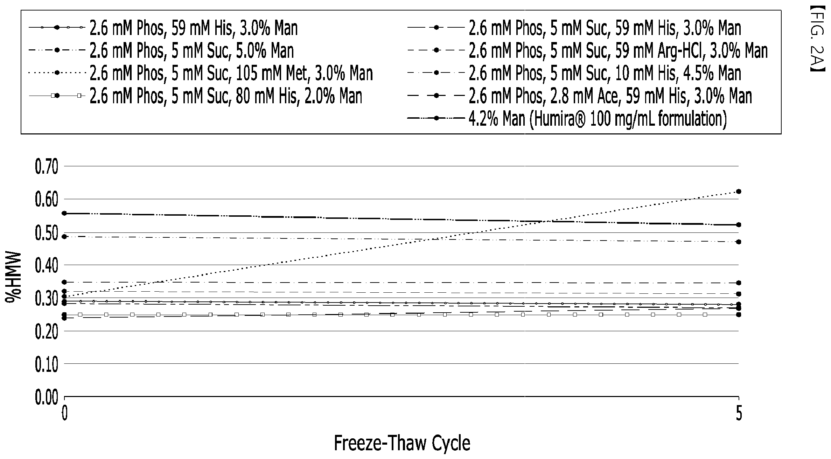

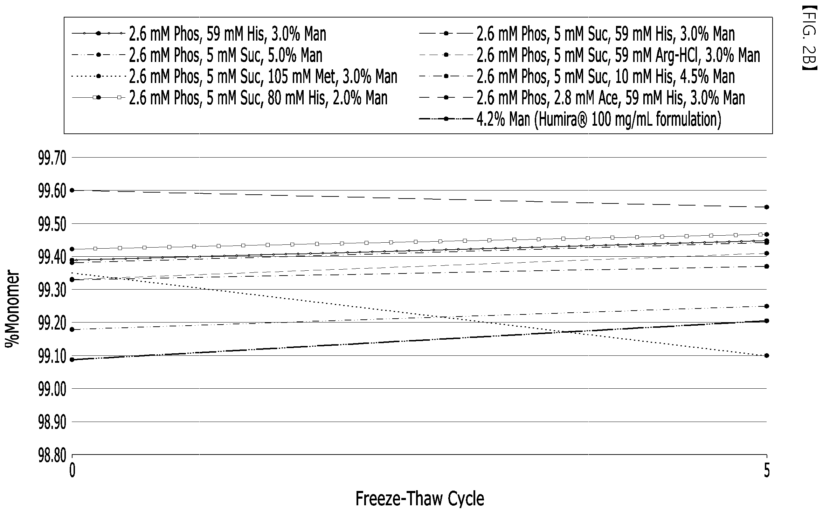

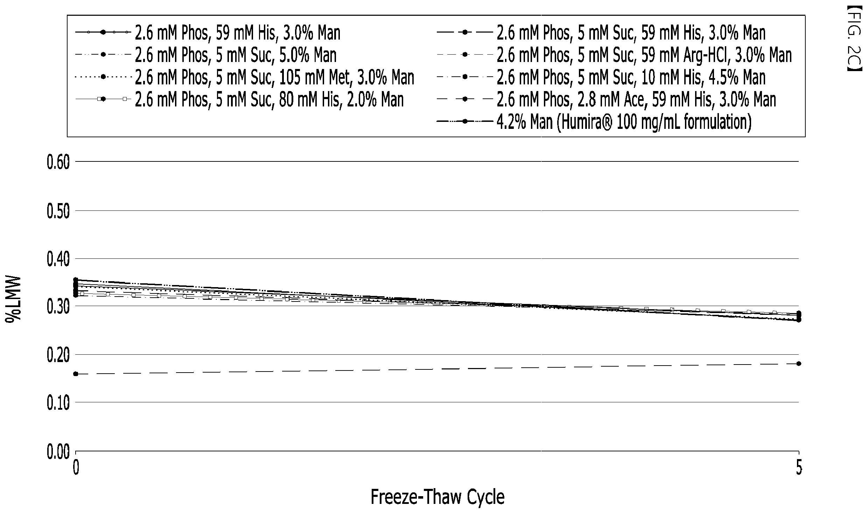

%HMW, %monomer, and %LMW of proteins in the formulations prepared in Example 1.1 were measured under freeze/thaw conditions (Freeze/thaw, 5 cycles; FT5; each cycle: 'frozen at -70 °C±10 °C for 18 hours or longer' + 'thawed at room temperature (25 °C) for 1 hour or longer') by SE-HPLC analysis according to Reference Example 1. The experiment was repeated three times (n=3) and the average values of the results are shown in the following Table 6, and FIGS. 2A to 2C.

[Table 6]

| %HMW, %Monomer, and %LMW under FT5 (Freeze/Thaw, 5 Cycles) | | | | | | | | | |
|---|---|---|---|---|---|---|---|---|---|
| Sample No. | %HMW | | | %Monomer | | | %LMW | | |
| | Initial | 5 Cyc. | Δ%H MW | Initial | 5 Cyc. | Δ%Mono mer | Initial | 5 Cyc. | Δ%L MW |
| 1 | 0.29 | 0.28 | -0.01 | 99.39 | 99.45 | 0.06 | 0.33 | 0.27 | -0.05 |
| SD | 0.01 | 0.02 | 0.01 | 0.03 | 0.03 | 0.02 | 0.02 | 0.04 | 0.02 |
| 2 | 0.28 | 0.27 | -0.01 | 99.38 | 99.44 | 0.06 | 0.33 | 0.28 | -0.05 |
| SD | 0.02 | 0.00 | 0.02 | 0.03 | 0.02 | 0.03 | 0.03 | 0.01 | 0.03 |
| 3 | 0.49 | 0.47 | -0.02 | 99.18 | 99.25 | 0.07 | 0.34 | 0.28 | -0.06 |
| SD | 0.01 | 0.01 | 0.01 | 0.02 | 0.01 | 0.01 | 0.01 | 0.01 | 0.00 |
| 4 | 0.32 | 0.31 | -0.01 | 99.33 | 99.41 | 0.08 | 0.35 | 0.27 | -0.07 |
| SD | 0.00 | 0.01 | 0.01 | 0.01 | 0.03 | 0.03 | 0.01 | 0.03 | 0.03 |
| 5 | 0.31 | 0.62 | 0.32 | 99.35 | 99.10 | -0.25 | 0.34 | 0.27 | -0.07 |
| SD | 0.01 | 0.02 | 0.01 | 0.01 | 0.01 | 0.02 | 0.00 | 0.02 | 0.02 |
| 6 | 0.35 | 0.35 | 0.00 | 99.33 | 99.37 | 0.04 | 0.32 | 0.28 | -0.04 |
| SD | 0.01 | 0.01 | 0.02 | 0.03 | 0.00 | 0.03 | 0.02 | 0.01 | 0.02 |
| 7 | 0.25 | 0.25 | 0.00 | 99.42 | 99.47 | 0.04 | 0.33 | 0.28 | -0.04 |
| SD | 0.02 | 0.01 | 0.01 | 0.04 | 0.02 | 0.05 | 0.02 | 0.02 | 0.04 |
| 8 | 0.24 | 0.27 | 0.04 | 99.60 | 99.55 | -0.05 | 0.16 | 0.18 | 0.02 |
| SD | 0.01 | 0.01 | 0.01 | 0.01 | 0.01 | 0.02 | 0.01 | 0.01 | 0.01 |
| 11 | 0.56 | 0.52 | -0.03 | 99.09 | 99.20 | 0.12 | 0.35 | 0.27 | -0.08 |
| SD | 0.01 | 0.01 | 0.01 | 0.02 | 0.02 | 0.03 | 0.03 | 0.02 | 0.04 |

The results of Examples 1.2 and 1.3 confirmed that the formulations including phosphate, phosphate+succinate, or phosphate+acetate as a buffer were superior in stability, as compared with the formulation including acetate (alone) or the buffer-free formulation not including a buffer having the same composition as a commercially available formulation Humira®100 mg/mL (pH 5.2, 4.2% mannitol, 0.1% PS 80). It was also confirmed that the formulations including histidine as an amino acid showed superior stability, as compared with the formulations including other amino acids or being free of amino acids.

Example 2. Stability under Photostress Conditions

2.1. Preparation of Formulation

To test a photostability effect of histidine on the antibody formulations, aqueous liquid formulations having the compositions of the following Table 7 were prepared using an anti-TNFa antibody (Adalimumab; anti-TNFa monoclonal antibody; CAS Registry Number: 331731-18-1) as a protein:

[Table 7]

| Sample No. | Protein Conc. (mg/mL) | pH | Buffer | Excipient 1 | Excipient 2 | Surfactant |
|---|---|---|---|---|---|---|
| 1 | 100 | 5.2 | 2.6 mM Na-phosphate | 59 mM His | 3.0% mannitol | 0.08% PS 20 |
| 2 | | | | | | |
| 3 | | | | N/A | 5.0% mannitol | |
| 4 | | | 2.6 mM Na-phosphate + 5 mM Na-succinate | 59 mM Arg-HCl | 3.0% mannitol | |
| 5 | | | | 10 mM His | 4.5% mannitol | |
| 6 | | | | 80 mM His | 2.0% mannitol | |
| 7* | | | N/A | N/A | 4.2% mannitol | 0.1% PS80 |
| [000204] (*: a sample including the same formulation ingredients as Humira® including 100 mg/mL of adalimumab; [000205] N/A: not applicable) | | | | | | |

2.2. Stability of Formulation under Photostress Conditions (%HMW, %Monomer, and %LMW)

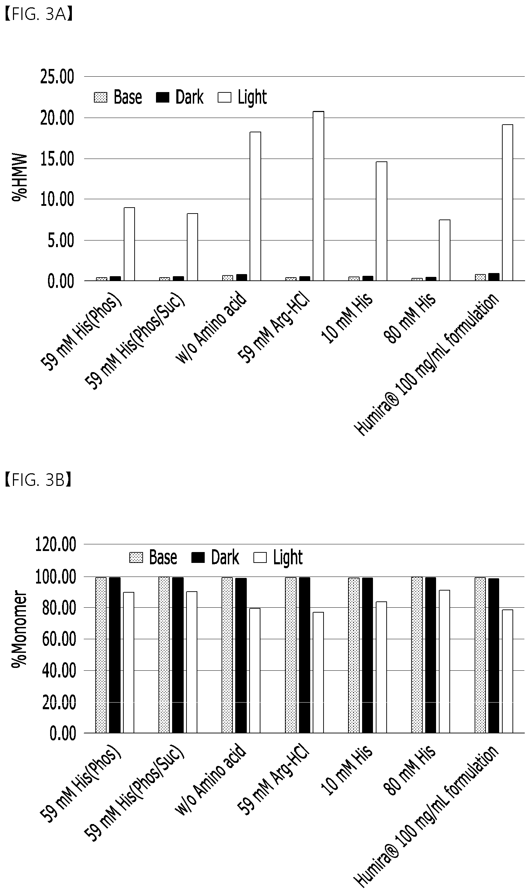

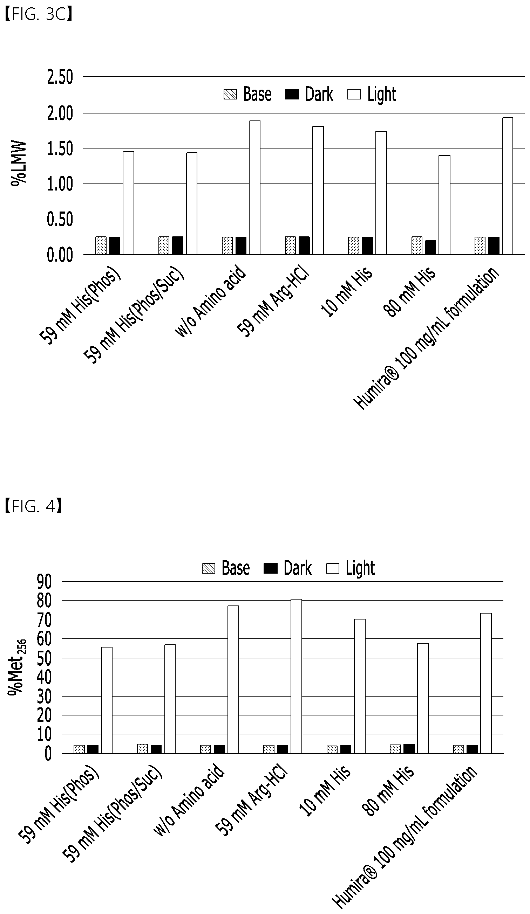

%HMW, %monomer, and %LMW of proteins in the formulations prepared in Example 2.1 were measured under photostress conditions of Reference Example 2 by SE-HPLC analysis according to Reference Example 1. The experiment was repeated three times (n=3) and the average values of the results are shown in the following Tables 8 to 10, and FIGS. 3A to 3C.

[Table 8]

| %HMW under Photostress Conditions | | | | | |
|---|---|---|---|---|---|
| Sample No. | %HMW | | | Δ%HMW | |
| | Base | Dark-control | Light-exposured | [Light - exposured %HMW] - [Base %HMW] | [Light-exposured %HMW] - [Dark-control %HMW] |
| 1 | 0.35 | 0.46 | 8.92 | 8.57 | 8.46 |
| SD | 0.01 | 0.02 | 0.15 | 0.14 | 0.13 |
| 2 | 0.33 | 0.43 | 8.19 | 7.86 | 7.76 |
| SD | 0.00 | 0.01 | 0.12 | 0.12 | 0.12 |
| 3 | 0.65 | 0.79 | 18.13 | 17.49 | 17.34 |
| SD | 0.02 | 0.03 | 0.61 | 0.60 | 0.58 |
| 4 | 0.40 | 0.50 | 20.79 | 20.39 | 20.29 |
| SD | 0.00 | 0.01 | 0.85 | 0.85 | 0.86 |
| 5 | 0.47 | 0.58 | 14.53 | 14.07 | 13.96 |
| SD | 0.01 | 0.01 | 0.34 | 0.34 | 0.34 |
| 6 | 0.31 | 0.40 | 7.45 | 7.14 | 7.05 |
| SD | 0.00 | 0.01 | 0.20 | 0.20 | 0.20 |
| 7 | 0.77 | 0.95 | 19.14 | 18.38 | 18.20 |
| SD | 0.01 | 0.01 | 0.26 | 0.26 | 0.26 |

[Table 9]

| %Monomer under Photostress Conditions | | | | | |
|---|---|---|---|---|---|
| Sample No. | %Monomer | | | Δ%Monomer | |
| | Base | Dark-control | Light-exposured | [Light - exposured %Monomer] - [Base %Monomer] | [Light-exposured %Monomer] - [Dark-control %Monomer] |
| 1 | 99.40 | 99.29 | 89.62 | -9.77 | -9.66 |
| SD | 0.01 | 0.02 | 0.17 | 0.16 | 0.15 |
| 2 | 99.42 | 99.31 | 90.37 | -9.05 | -8.94 |
| SD | 0.00 | 0.01 | 0.11 | 0.11 | 0.12 |
| 3 | 99.10 | 98.95 | 79.97 | -19.13 | -18.98 |
| SD | 0.01 | 0.03 | 0.62 | 0.61 | 0.60 |
| 4 | 99.35 | 99.25 | 77.40 | -21.95 | -21.85 |
| SD | 0.00 | 0.01 | 0.83 | 0.83 | 0.84 |
| 5 | 99.28 | 99.17 | 83.73 | -15.56 | -15.44 |
| SD | 0.01 | 0.02 | 0.35 | 0.34 | 0.35 |
| 6 | 99.44 | 99.40 | 91.15 | -8.29 | -8.25 |
| SD | 0.02 | 0.11 | 0.23 | 0.25 | 0.30 |
| 7 | 98.98 | 98.80 | 78.93 | -20.05 | -19.88 |
| SD | 0.01 | 0.01 | 0.24 | 0.24 | 0.24 |

[Table 10]

| %LMW under Photostress Conditions | | | | | |
|---|---|---|---|---|---|
| Sample No. | %LMW | | | Δ%LMW | |
| | Base | Dark-control | Light-exposured | [Light - exposured %LMW] - [Base %LMW] | [Light - exposured %LMW] - [Dark-control %LMW] |
| 1 | 0.25 | 0.25 | 1.46 | 1.21 | 1.21 |
| SD | 0.00 | 0.00 | 0.02 | 0.02 | 0.02 |
| 2 | 0.25 | 0.25 | 1.44 | 1.19 | 1.19 |
| SD | 0.00 | 0.01 | 0.02 | 0.02 | 0.02 |
| 3 | 0.25 | 0.26 | 1.89 | 1.64 | 1.64 |
| SD | 0.01 | 0.01 | 0.02 | 0.03 | 0.03 |
| 4 | 0.25 | 0.26 | 1.81 | 1.56 | 1.55 |
| SD | 0.00 | 0.01 | 0.02 | 0.02 | 0.02 |
| 5 | 0.25 | 0.26 | 1.74 | 1.49 | 1.48 |
| SD | 0.00 | 0.01 | 0.02 | 0.02 | 0.02 |
| 6 | 0.25 | 0.20 | 1.40 | 1.15 | 1.20 |
| SD | 0.01 | 0.10 | 0.03 | 0.04 | 0.11 |
| 7 | 0.25 | 0.25 | 1.93 | 1.68 | 1.68 |
| SD | 0.01 | 0.00 | 0.03 | 0.03 | 0.03 |

As confirmed from the above results, the %HMW and variations of %HMW under the photostress conditions were observed in this order of Arg-HCl > Amino acid-free > His, indicating that the histidine-containing formulations are of higher photostability than the amino acid-free formulations or the Arg-HCl-containing formulations. The histidine-containing formulations exhibited remarkably excellent photostability, as compared with the amino acid-free formulations having the same composition as the commercially available formulation Humira®100 mg/mL (pH 5.2, 4.2% mannitol, 0.1% PS 80). In addition, %HMW and variation of %HMW according to His concentrations under the photostress conditions were observed in this order of 10 mM His > 59 mM His = 80 mM His, indicating that formulations containing 59 mM to 80 mM His were of higher photostability than those containing 10 mM His, demonstrating that a His concentration of 59 mM or more guarantees higher photostability. In addition, the results of %Monomer and %LMW also showed that the histidine-containing formulations (Sample Nos. 1, 2, 5, and 6) are of higher photostability than formulations free of amino acids or formulation containing an amino acid (e.g., arginine) other than histidine.

2.3. Amino Acid Oxidation Rate under Photostress Conditions

For the formulations prepared in Example 2.1, oxidation rates ($\%Met_{256/light\text{-}exposed}$) of $Met_{256}$ (methionine at position 256) in the protein were measured under light-exposed conditions of Reference Example 2 with reference to Reference Example 3. The experiment was repeated three times (n=3) and the average values of the results are given in the following Table 11 and FIG. 4.

[Table 11]

| Amino Acid ($Met_{256}$) Oxidation Rate ($\%Met_{256}$) and Amino Acid Oxidation Rate Variation ($\Delta\%Met_{256}$ = Light-exposed %Met256 - Base %Met256) under Photostress Conditions | | | | | |
|---|---|---|---|---|---|
| Sample No. | %Met256 Base | $\Delta$%Met256 Dark-control | Light-exposed | [Light - exposed $\%Met_{256}$] - [Base %Met256] | [Light - exposed $\%Met_{256}$] - [Dark-control %Met256] |
| 1 | 4.23 | 4.43 | 55.80 | 51.57 | 51.37 |
| SD | 0.06 | 0.31 | 1.04 | 1.08 | 1.34 |
| 2 | 4.67 | 4.57 | 57.07 | 52.40 | 52.50 |
| SD | 0.42 | 0.32 | 0.49 | 0.10 | 0.66 |
| 3 | 4.37 | 4.57 | 77.77 | 73.40 | 73.20 |
| SD | 0.12 | 0.40 | 2.99 | 3.04 | 3.35 |
| 4 | 4.23 | 4.57 | 80.83 | 76.60 | 76.27 |
| SD | 0.21 | 0.32 | 4.03 | 3.82 | 3.72 |
| 5 | 4.07 | 4.57 | 70.23 | 66.17 | 65.67 |
| SD | 0.21 | 0.25 | 0.35 | 0.38 | 0.31 |
| 6 | 4.20 | 4.80 | 57.63 | 53.43 | 52.83 |
| SD | 0.26 | 0.46 | 3.51 | 3.62 | 3.88 |
| 7 | 4.13 | 4.43 | 73.27 | 69.13 | 68.83 |
| SD | 0.21 | 0.35 | 2.11 | 1.92 | 1.76 |

As confirmed from the results, $Met_{256}$ oxidation rate variations under the photostress conditions were observed in this order of Arg-HCl > Amino acid-free > His, indicating that histidine-containing formulations were more suitable in terms of photostability than amino acid-free formulations or Arg-HCl-containing formulations. It was observed that the histidine-containing formulations showed noticeably low amino acid ($Met_{256}$) oxidation rates and variation thereof, and thus had remarkably high photostability, as compared with the amino acid-free formulations that have the same composition as the commercially available Humira®100 mg/mL (pH 5.2, 4.2% mannitol, 0.1% PS 80).

In addition, amino acid oxidation rate variations according to histidine concentrations were measured, and as a result, observed in this order of 10 mM His > 59 mM His = 80 mM His, indicating that a His concentration of 59 mM or more guarantees excellent photostability in the formulation.

Taken together, it was demonstrated that histidine has an antioxidant activity. Particularly, when the content of histidine in the antibody formulation was 10 mM or more and 80 mM or less (e.g., about 59 mM), histidine exhibited the highest stability and antioxidant activity.

Example 3. Stability Depending on Type of Polyol

In the antibody formulation of the present disclosure, the stability depending on the type of polyol was tested, and to select optimal polyols, aqueous liquid formulations having the compositions in the following Table 12 were prepared using the anti-TNFa antibody (Adalimumab; anti-TNFa monoclonal antibody; CAS Registry Number: 331731-18-1) as a protein:

[Table 12]

| No. | Protein Conc. (mg/mL) | pH | Buffer | Excipient 1 | Excipient 2 | Surfactant |
|---|---|---|---|---|---|---|
| 1 | | | 2.6 mM Na-phosphate | 59 mM histidine | 3.0% mannitol | 0.08% PS 20 |
| 2 | | | 2.6 mM Na-phosphate + 5 mM Na-succinate | | | |
| 3 | 100 | 5.2 | 2.6 mM Na-phosphate + 5 mM Na-succinate | 59 mM histidine | 2.0% mannitol | 0.08% PS 20 |
| 4 | | | | | 5.0% mannitol | |
| 5 | | | | | N/A | |
| 6 | | | | | 3.0% sucrose | |
| 7 | | | | | 80 mM NaCl | |

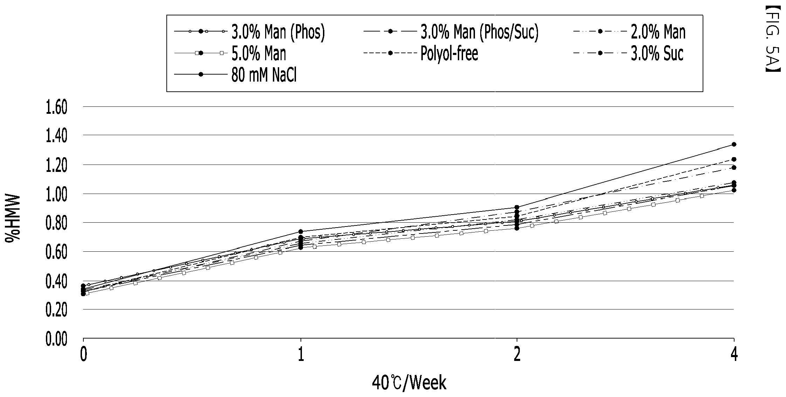

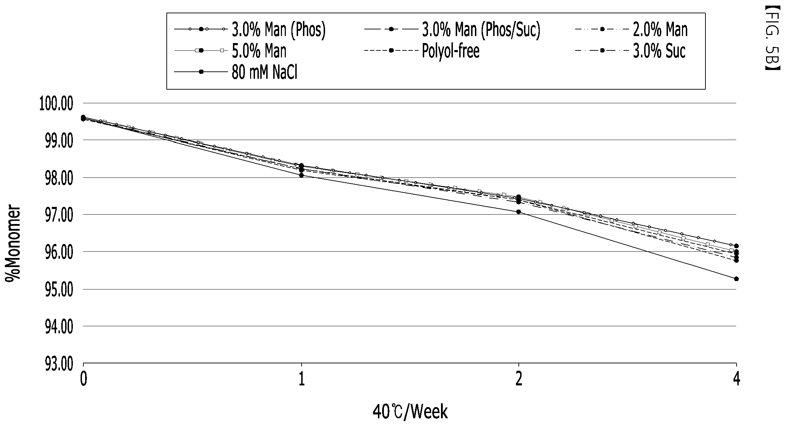

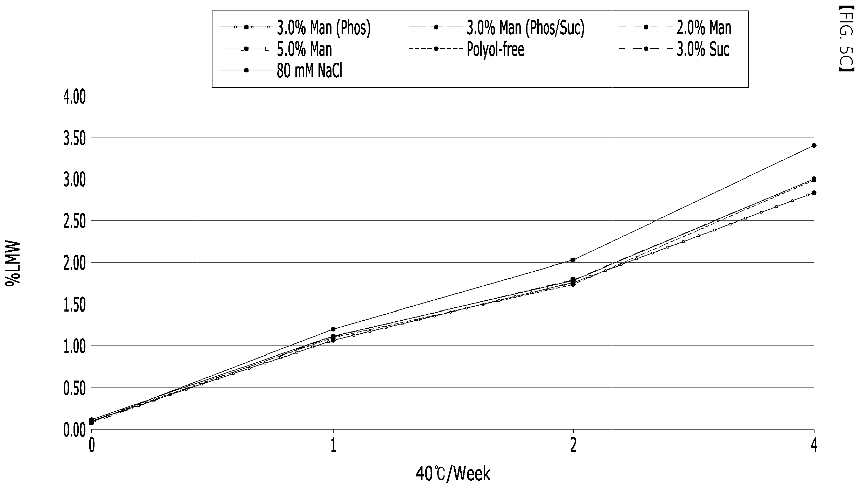

The prepared formulations were stored for four weeks under the heat stress conditions of 40±2 °C and 75±5% RH, and then %HMW, %monomer, and %LMW of the proteins included in the formulations were measured by SE-HPLC analysis according to Reference Example 1. The experiment was repeated three times (n=3) and the average values of the results are shown in the following Tables 13 to 15 and FIGS. 5A to 5C.

[Table 13]

| Polyol | Sample No. | %HMW | | | | |
|---|---|---|---|---|---|---|
| | | Initial | 1 Wk | 2 Wk | 4 Wk | Δ%HMW (4wk %HMW - initial %HMW) |
| 3.0% mannitol (phosphate) | 1 | 0.36 | 0.65 | 0.81 | 1.05 | 0.69 |
| | SD | 0.01 | 0.01 | 0.00 | 0.03 | 0.03 |
| 3.0% mannitol (phosphate/ succinate) | 2 | 0.34 | 0.64 | 0.79 | 1.05 | 0.71 |
| | SD | 0.01 | 0.01 | 0.01 | 0.03 | 0.02 |
| 2.0% mannitol | 3 | 0.34 | 0.66 | 0.82 | 1.07 | 0.73 |
| | SD | 0.01 | 0.03 | 0.03 | 0.04 | 0.04 |
| 5.0% mannitol | 4 | 0.31 | 0.62 | 0.76 | 1.02 | 0.71 |
| | SD | 0.01 | 0.01 | 0.03 | 0.02 | 0.02 |
| Polyol-free | 5 | 0.34 | 0.70 | 0.85 | 1.24 | 0.90 |
| | SD | 0.02 | 0.02 | 0.02 | 0.14 | 0.16 |
| 3.0% sucrose | 6 | 0.33 | 0.67 | 0.87 | 1.18 | 0.84 |
| | SD | 0.01 | 0.01 | 0.05 | 0.14 | 0.14 |

(continued)

| Polyol | Sample No. | %HMW | | | | |
| --- | --- | --- | --- | --- | --- | --- |
| | | Initial | 1 Wk | 2 Wk | 4 Wk | Δ%HMW (4wk %HMW - initial %HMW) |
| 80 mM NaCl | 7 | 0.34 | 0.73 | 0.90 | 1.34 | 1.00 |
| | SD | 0.01 | 0.03 | 0.03 | 0.04 | 0.04 |

[Table 14]

| Polyol | Sample No. | %Monomer | | | | |
| --- | --- | --- | --- | --- | --- | --- |
| | | Initial | 1 Wk | 2 Wk | 4 Wk | Δ%Monomer (4wk %Monomer initial %Monomer) |
| 3.0% mannitol | 1 | 99.57 | 98.29 | 97.41 | 96.13 | -3.44 |
| (phosphate) | SD | 0.03 | 0.02 | 0.01 | 0.06 | 0.04 |
| 3.0% mannitol (phosphate/ succinate) | 2 | 99.56 | 98.25 | 97.46 | 95.95 | -3.61 |
| | SD | 0.04 | 0.01 | 0.02 | 0.03 | 0.02 |
| 2.0% mannitol | 3 | 99.57 | 98.22 | 97.40 | 95.93 | -3.64 |
| | SD | 0.03 | 0.03 | 0.04 | 0.02 | 0.01 |
| 5.0% mannitol | 4 | 99.60 | 98.26 | 97.46 | 95.99 | -3.61 |
| | SD | 0.00 | 0.02 | 0.04 | 0.02 | 0.02 |
| Polyol-free | 5 | 99.58 | 98.18 | 97.40 | 95.77 | -3.81 |
| | SD | 0.03 | 0.03 | 0.01 | 0.16 | 0.18 |
| 3.0% sucrose | 6 | 99.59 | 98.22 | 97.34 | 95.82 | -3.77 |
| | SD | 0.02 | 0.02 | 0.06 | 0.16 | 0.17 |
| 80 mM NaCl | 7 | 99.58 | 98.06 | 97.07 | 95.26 | -4.32 |
| | SD | 0.02 | 0.04 | 0.02 | 0.05 | 0.06 |

[Table 15]

| Polyol | Sample No. | %LMW | | | | |
| --- | --- | --- | --- | --- | --- | --- |
| | | Initial | 1 Wk | 2 Wk | 4 Wk | Δ%LMW (4wk %LMW - initial %LMW) |
| 3.0% mannitol (phosphate) | 1 | 0.08 | 1.06 | 1.78 | 2.83 | 2.75 |
| | SD | 0.03 | 0.01 | 0.01 | 0.03 | 0.02 |
| 3.0% mannitol (phosphate/ succinate) | 2 | 0.10 | 1.12 | 1.76 | 3.00 | 2.90 |
| | SD | 0.03 | 0.01 | 0.03 | 0.02 | 0.02 |
| 2.0% mannitol | 3 | 0.09 | 1.12 | 1.77 | 3.00 | 2.91 |
| | SD | 0.02 | 0.01 | 0.01 | 0.03 | 0.05 |
| 5.0% mannitol | 4 | 0.08 | 1.12 | 1.78 | 2.99 | 2.91 |
| | SD | 0.00 | 0.01 | 0.01 | 0.02 | 0.02 |
| Polyol-free | 5 | 0.08 | 1.12 | 1.75 | 2.99 | 2.91 |
| | SD | 0.01 | 0.02 | 0.01 | 0.02 | 0.03 |

(continued)

| 3.0% sucrose | 6 | 0.07 | 1.11 | 1.79 | 3.00 | 2.93 |
| | SD | 0.03 | 0.02 | 0.02 | 0.02 | 0.04 |
| 80 mM NaCl | 7 | 0.08 | 1.20 | 2.02 | 3.40 | 3.32 |
| | SD | 0.01 | 0.01 | 0.02 | 0.02 | 0.02 |

As shown in the results, when given mannitol as the polyol, both the formulations containing phosphate or phosphate+succinate as a buffer showed remarkably lower %HMW at week 4 and remarkably lower %HMW variation over four weeks under the heat stress conditions to be of higher stability than any of the polyol-free, sucrose-containing, and NaCl-containing formulations. No significant difference in stability was observed according to contents of mannitol.

**Claims**

1. A pharmaceutical composition comprising:

   40 mg/ml to 200 mg/ml of adalimumab;
   a buffer comprising phosphate; and
   histidine,
   wherein pH is 5 to 7, and
   wherein the composition is free of citrate, free of pharmaceutically acceptable salts thereof, or free of both of them, and
   is free of salts other than pharmaceutically acceptable salts of phosphate, succinate, and acetate.

2. The pharmaceutical composition of claim 1, wherein the composition is free of amino acids other than histidine.

3. The pharmaceutical composition of claim 1 or 2, wherein the composition is free of ethylenediaminetetraacetic acid (EDTA).

4. The pharmaceutical composition of any one of claims 1 to 3, wherein the salts other than pharmaceutically acceptable salts of phosphate, succinate, and acetate are one or more selected from the group consisting of sodium chloride, sodium sulfate, and potassium chloride.

5. The pharmaceutical composition of any one of claims 1 to 4, wherein the buffer further comprises one or more selected from the group consisting of succinate and acetate.

6. The pharmaceutical composition of any one of claims 1 to 5, wherein a concentration of the buffer is 0.5 mM to 50 mM.

7. The pharmaceutical composition of any one of claims 1 to 6, wherein the buffer comprises

   (1) 1.7 mM to 10 mM of phosphate or a pharmaceutically acceptable salt thereof, or
   (2) (i) 1.7 mM to 10 mM of phosphate or a pharmaceutically acceptable salt thereof, and (ii) one or more selected from the group consisting of 4.1 mM to 12 mM of succinate, 1.7 mM to 10 mM of acetate, and pharmaceutically acceptable salts thereof.

8. The pharmaceutical composition of any one of claims 1 to 7, wherein a concentration of histidine is 40 mM to 80 mM.

9. The pharmaceutical composition of any one of claims 1 to 8, wherein the composition further comprises a polyol.

10. The pharmaceutical composition of claim 9, wherein the polyol comprises one or more selected from the group consisting of mannitol, sorbitol, sucrose, trehalose, maltose, lactose, xylitol, arabitol, meglumine, erythritol, lactitol, maltitol, and inositol.

11. The pharmaceutical composition of claim 9 or 10, wherein a concentration of the polyol is 2 %(w/v) to 10 %(w/v).

**12.** The pharmaceutical composition of any one of claims 9 to 11, wherein the polyol is mannitol.

**13.** The pharmaceutical composition of claim 12, wherein a concentration of the mannitol is 2 %(w/v) to 5 %(w/v).

**14.** The pharmaceutical composition of any one of claims 1 to 13, wherein the composition further comprises a surfactant.

**15.** The pharmaceutical composition of claim 14, wherein the surfactant comprises polysorbate 20, polysorbate 80, or a combination thereof.

**16.** The pharmaceutical composition of claim 15, wherein the surfactant is polysorbate 20.

**17.** The pharmaceutical composition of claim 16, wherein a concentration of polysorbate 20 is 0.01 %(w/v) to 0.9 %(w/v).

**18.** The pharmaceutical composition of any one of claims 1 to 17, wherein a concentration of the adalimumab is 40 mg/ml to 100 mg/ml.

**19.** A pharmaceutical composition comprising:

40 mg/ml to 200 mg/ml of adalimumab;
a buffer comprising phosphate; and
histidine,
wherein pH is 5 to 7,
wherein the composition is free of citrate, free of pharmaceutically acceptable salts thereof, or free of both of them, and
is free of salts other than pharmaceutically acceptable salts of phosphate, succinate, and acetate; and
an aggregation rate of the adalimumab is about 15 % or less under light exposure conditions.

**20.** The pharmaceutical composition of claim 19, wherein a variation ($\Delta\%Met_{256}$) of an oxidation rate ($\%Met_{256/light\text{-}exposured}$) of methionine residues of the adalimumab under light exposure conditions is 68 % or less.

**21.** The pharmaceutical composition of claim 19 or 20,
wherein the composition is free of amino acids other than histidine.

**22.** The pharmaceutical composition of any one of claims 19 to 21,
wherein the composition is free of ethylenediaminetetraacetic acid (EDTA).

**23.** The pharmaceutical composition of any one of claims 19 to 22, wherein the salts other than pharmaceutically acceptable salts of phosphate, succinate, and acetate are one or more selected from the group consisting of sodium chloride, sodium sulfate, and potassium chloride.

**24.** The pharmaceutical composition of any one of claims 19 to 23, wherein the buffer further comprises one or more selected from the group consisting of succinate and acetate.

**25.** The pharmaceutical composition of any one of claims 19 to 24, wherein a concentration of the buffer is 0.5 mM to 50 mM.

**26.** The pharmaceutical composition of any one of claims 19 to 25, wherein the buffer comprises:

(1) 1.7 mM to 10 mM of phosphate or a pharmaceutically acceptable salt thereof, or
(2) (i) 1.7 mM to 10 mM of phosphate or a pharmaceutically acceptable salt thereof, and (ii) one or more selected from the group consisting of 4.1 mM to 12 mM of succinate, 1.7 mM to 10 mM of acetate, and pharmaceutically acceptable salts thereof.

**27.** The pharmaceutical composition of any one of claims 19 to 26, wherein a concentration of histidine is 40 mM to 80 mM.

**28.** The pharmaceutical composition of any one of claims 19 to 27, wherein the composition further comprises a polyol.

**29.** The pharmaceutical composition of claim 28, wherein the polyol comprises one or more selected from the group consisting of mannitol, sorbitol, sucrose, trehalose, maltose, lactose, xylitol, arabitol, meglumine, erythritol, lactitol, maltitol, and inositol.

30. The pharmaceutical composition of claim 28 or 29, wherein a concentration of the polyol is 2 %(w/v) to 10 %(w/v).

31. The pharmaceutical composition of any one of claims 28 to 30, wherein the polyol is mannitol.

32. The pharmaceutical composition of claim 31, wherein a concentration of the mannitol is 2 %(w/v) to 5 %(w/v).

33. The pharmaceutical composition of any one of claims 19 to 32, wherein the composition further comprises a surfactant.

34. The pharmaceutical composition of claim 33, wherein the surfactant comprises polysorbate 20, polysorbate 80, or a combination thereof.

35. The pharmaceutical composition of claim 33, wherein the surfactant is polysorbate 20.

36. The pharmaceutical composition of claim 35, wherein a concentration of the polysorbate 20 is 0.01 %(w/v) to 0.9 %(w/v).

37. The pharmaceutical composition of any one of claims 19 to 36, wherein a concentration of the adalimumab is 50 mg/ml to 100 mg/ml.

38. A device comprising the pharmaceutical composition of any one of claims 1 to 37.

39. The device of claim 38, wherein the device is for intravenous or subcutaneous administration of the pharmaceutical composition.

40. The device of claim 38 or 39, comprising the pharmaceutical composition in a container selected from the group consisting of a syringe, a pre-filled syringe, an auto-injector, a bottle, a vial, and a tube.

41. A method of treating a patient, the method comprising administering the pharmaceutical composition of any one of claims 1 to 37 to the patient in need of administration of the antibody.

Amended claims under Art. 19.1 PCT

1. A pharmaceutical composition comprising:

    100 mg/ml of adalimumab;
    a buffer comprising phosphate; and
    histidine,
    wherein pH is 5 to 7, and
    wherein the composition is free of citrate, free of pharmaceutically acceptable salts thereof, or free of both of them, and
    is free of salts other than pharmaceutically acceptable salts of phosphate, succinate, and acetate.

2. The pharmaceutical composition of claim 1, wherein the composition is free of amino acids other than histidine.

3. The pharmaceutical composition of claim 1 or 2, wherein the composition is free of ethylenediaminetetraacetic acid (EDTA).

4. The pharmaceutical composition of any one of claims 1 to 3, wherein the salts other than pharmaceutically acceptable salts of phosphate, succinate, and acetate are one or more selected from the group consisting of sodium chloride, sodium sulfate, and potassium chloride.

5. The pharmaceutical composition of any one of claims 1 to 4, wherein the buffer further comprises one or more selected from the group consisting of succinate and acetate.

6. The pharmaceutical composition of any one of claims 1 to 5, wherein a concentration of the buffer is 0.5 mM to 50 mM.

7. The pharmaceutical composition of any one of claims 1 to 6, wherein the buffer comprises

    (1) 1.7 mM to 10 mM of phosphate or a pharmaceutically acceptable salt thereof, or
    (2) (i) 1.7 mM to 10 mM of phosphate or a pharmaceutically acceptable salt thereof, and (ii) one or more selected from the group consisting of 4.1 mM to 12 mM of succinate, 1.7 mM to 10 mM of acetate, and pharmaceutically acceptable salts thereof.

8. The pharmaceutical composition of any one of claims 1 to 7, wherein a concentration of histidine is 40 mM to 80 mM.

9. The pharmaceutical composition of any one of claims 1 to 8, wherein the composition further comprises a polyol.

10. The pharmaceutical composition of claim 9, wherein the polyol comprises one or more selected from the group consisting of mannitol, sorbitol, sucrose, trehalose, maltose, lactose, xylitol, arabitol, meglumine, erythritol, lactitol, maltitol, and inositol.

11. The pharmaceutical composition of claim 9 or 10, wherein a concentration of the polyol is 2 %(w/v) to 10 %(w/v).

12. The pharmaceutical composition of any one of claims 9 to 11, wherein the polyol is mannitol.

13. The pharmaceutical composition of claim 12, wherein a concentration of the mannitol is 2 %(w/v) to 5 %(w/v).

14. The pharmaceutical composition of any one of claims 1 to 13, wherein the composition further comprises a surfactant.

15. The pharmaceutical composition of claim 14, wherein the surfactant comprises polysorbate 20, polysorbate 80, or a combination thereof.

16. The pharmaceutical composition of claim 15, wherein the surfactant is polysorbate 20.

17. The pharmaceutical composition of claim 16, wherein a concentration of polysorbate 20 is 0.01 %(w/v) to 0.9 %(w/v).

18.

19. A pharmaceutical composition comprising:

    100 mg/ ml of adalimumab;
    a buffer comprising phosphate; and
    histidine,
    wherein pH is 5 to 7,
    wherein the composition is free of citrate, free of pharmaceutically acceptable salts thereof, or free of both of them, and
    is free of salts other than pharmaceutically acceptable salts of phosphate, succinate, and acetate; and
    an aggregation rate of the adalimumab is about 15 % or less under light exposure conditions.

20. The pharmaceutical composition of claim 19, wherein a variation ($\Delta\%Met_{256}$) of an oxidation rate ($\%Met_{256/light\text{-}exposured}$) of methionine residues of the adalimumab under light exposure conditions is 68 % or less.

21. The pharmaceutical composition of claim 19 or 20, wherein the composition is free of amino acids other than histidine.

22. The pharmaceutical composition of any one of claims 19 to 21, wherein the composition is free of ethylenediaminetetraacetic acid (EDTA).

23. The pharmaceutical composition of any one of claims 19 to 22, wherein the salts other than pharmaceutically acceptable salts of phosphate, succinate, and acetate are one or more selected from the group consisting of sodium chloride, sodium sulfate, and potassium chloride.

24. The pharmaceutical composition of any one of claims 19 to 23, wherein the buffer further comprises one or more selected from the group consisting of succinate and acetate.

25. The pharmaceutical composition of any one of claims 19 to 24, wherein a concentration of the buffer is 0.5 mM to 50 mM.

26. The pharmaceutical composition of any one of claims 19 to 25, wherein the buffer comprises:

   (1) 1.7 mM to 10 mM of phosphate or a pharmaceutically acceptable salt thereof, or
   (2) (i) 1.7 mM to 10 mM of phosphate or a pharmaceutically acceptable salt thereof, and (ii) one or more selected from the group consisting of 4.1 mM to 12 mM of succinate, 1.7 mM to 10 mM of acetate, and pharmaceutically acceptable salts thereof.

27. The pharmaceutical composition of any one of claims 19 to 26, wherein a concentration of histidine is 40 mM to 80 mM.

28. The pharmaceutical composition of any one of claims 19 to 27, wherein the composition further comprises a polyol.

29. The pharmaceutical composition of claim 28, wherein the polyol comprises one or more selected from the group consisting of mannitol, sorbitol, sucrose, trehalose, maltose, lactose, xylitol, arabitol, meglumine, erythritol, lactitol, maltitol, and inositol.

30. The pharmaceutical composition of claim 28 or 29, wherein a concentration of the polyol is 2 %(w/v) to 10 %(w/v).

31. The pharmaceutical composition of any one of claims 28 to 30, wherein the polyol is mannitol.

32. The pharmaceutical composition of claim 31, wherein a concentration of the mannitol is 2 %(w/v) to 5 %(w/v).

33. The pharmaceutical composition of any one of claims 19 to 32, wherein the composition further comprises a surfactant.

34. The pharmaceutical composition of claim 33, wherein the surfactant comprises polysorbate 20, polysorbate 80, or a combination thereof.

35. The pharmaceutical composition of claim 33, wherein the surfactant is polysorbate 20.

36. The pharmaceutical composition of claim 35, wherein a concentration of the polysorbate 20 is 0.01 %(w/v) to 0.9 %(w/v).

37.

38. A device comprising the pharmaceutical composition of any one of claims 1 to 17 and claims 19 to 36.

39. The device of claim 38, wherein the device is for intravenous or subcutaneous administration of the pharmaceutical composition.

40. The device of claim 38 or 39, comprising the pharmaceutical composition in a container selected from the group consisting of a syringe, a pre-filled syringe, an auto-injector, a bottle, a vial, and a tube.

41. A method of treating a patient, the method comprising administering the pharmaceutical composition of any one of claims 1 to 17 and claims 19 to 36 to the patient in need of administration of the antibody.

【FIG. 1A】

【FIG. 1B】

【FIG. 1C】

【FIG. 2A】

【FIG. 2B】

[FIG. 2C]

EP 3 909 607 A1

【FIG. 3A】

【FIG. 3B】

【FIG. 3C】

【FIG. 4】

【FIG. 5A】

【FIG. 5B】

【FIG. 5C】

## INTERNATIONAL SEARCH REPORT

| International application No. |
|---|
| PCT/KR2020/000611 |

A. CLASSIFICATION OF SUBJECT MATTER

*A61K 39/395(2006.01)i, A61K 47/02(2006.01)i, A61K 47/22(2006.01)i, A61K 47/14(2006.01)i, A61K 47/26(2006.01)i, A61P 19/02(2006.01)i, A61P 29/00(2006.01)i, C07K 16/24(2006.01)i, A61K 39/00(2006.01)i*

According to International Patent Classification (IPC) or to both national classification and IPC

B. FIELDS SEARCHED

Minimum documentation searched (classification system followed by classification symbols)

A61K 39/395; A61K 47/10; A61K 47/22; A61K 47/26; A61K 9/00; C07K 16/24; A61K 47/02; A61K 47/14; A61P 19/02; A61P 29/00; A61K 39/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)

eKOMPASS (KIPO internal) & Keywords: adalimumab, phosphate, histidine, citrate, succinate, acetate

C. DOCUMENTS CONSIDERED TO BE RELEVANT

| Category* | Citation of document, with indication, where appropriate, of the relevant passages | Relevant to claim No. |
|---|---|---|
| X | US 2014-0186361 A1 (COHERUS BIOSCIENCES, INC.) 03 July 2014<br>See claims 1-7, 17-19, 24, 29-38, 42; paragraphs [0009]-[0014]. | 1-3,19-21 |
| Y | US 2017-0189527 A1 (ARES TRADING SA.) 06 July 2017<br>See claims 1-8, 14-16. | 1-3,19-21 |
| Y | WO 2017-136433 A1 (ONCOBIOLOGICS, INC.) 10 August 2017<br>See abstract; claims 1-14. | 1-3,19-21 |
| A | EP 2946765 A1 (ARES TRADING S.A.) 25 November 2015<br>See the entire document. | 1-3,19-21 |
| A | US 2017-0252437 A1 (RICHTER GEDEON NYRT.) 07 September 2017<br>See the entire document. | 1-3,19-21 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01 MAY 2020 (01.05.2020) | 01 MAY 2020 (01.05.2020) |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | |
| | Telephone No. |

Form PCT/ISA/210 (second sheet) (January 2015)

## INTERNATIONAL SEARCH REPORT

International application No.

**PCT/KR2020/000611**

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 41

because they relate to subject matter not required to be searched by this Authority, namely:

Claim 41 pertains to a method for treatment of the human body by surgery or therapy, and thus pertains to a subject matter on which the International Searching Authority is not required to carry out an international search under the provisions of PCT Article 17(2)(a)(i) and PCT Rule 39.1(iv).

2. ☒ Claims Nos.: 10, 13, 15-17, 29, 32, 34-36, 39

because they relate to parts of the international application that do not comply with the prescribed requirements to such an extent that no meaningful international search can be carried out, specifically:

Claims 10, 13, 15-17, 29, 32, 34-36 and 39 refer to multiple dependent claims not meeting the requirement of PCT Rule 6.4 (a).

3. ☒ Claims Nos.: 4-9, 11-12, 14, 18, 22-28, 30-31, 33, 37-38, 40-41

because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.

**PCT/KR2020/000611**

| Patent document cited in search report | Publication date | Patent family member | Publication date |
|---|---|---|---|
| US 2014-0186361 A1 | 03/07/2014 | AR 092470 A1 | 22/04/2015 |
| | | AU 2013-312300 A1 | 16/04/2015 |
| | | BR 112015004984 A2 | 04/07/2017 |
| | | CA 2884182 A1 | 13/03/2014 |
| | | CL 2015000574 A1 | 23/10/2015 |
| | | CN 104768576 A | 08/07/2015 |
| | | EA 201590518 A1 | 31/05/2016 |
| | | EA 201791717 A1 | 30/03/2018 |
| | | EP 2892550 A2 | 15/07/2015 |
| | | EP 2892550 B1 | 18/12/2019 |
| | | HK 1210601 A1 | 29/04/2016 |
| | | IL 237583 A | 30/04/2015 |
| | | JP 2015-527402 A | 17/09/2015 |
| | | JP 2019-069993 A | 09/05/2019 |
| | | JP 6463268 B2 | 30/01/2019 |
| | | KR 10-2015-0047140 A | 04/05/2015 |
| | | MX 2015003007 A | 05/06/2015 |
| | | SG 10201705668 A | 30/08/2017 |
| | | TW 201424749 A | 01/07/2014 |
| | | US 2015-0182626 A1 | 02/07/2015 |
| | | US 2015-0190512 A1 | 09/07/2015 |
| | | US 2016-0031982 A1 | 04/02/2016 |
| | | US 2016-0263226 A1 | 15/09/2016 |
| | | US 2016-0303233 A1 | 20/10/2016 |
| | | US 2017-0312361 A1 | 02/11/2017 |
| | | US 2018-0021433 A1 | 25/01/2018 |
| | | US 2018-0043018 A1 | 15/02/2018 |
| | | US 2019-0070294 A1 | 07/03/2019 |
| | | US 9340611 B2 | 17/05/2016 |
| | | US 9682145 B2 | 20/06/2017 |
| | | US 9707293 B2 | 18/07/2017 |
| | | US 9731009 B2 | 15/08/2017 |
| | | US 9782480 B2 | 10/10/2017 |
| | | US 9808525 B2 | 07/11/2017 |
| | | US 9861695 B2 | 09/01/2018 |
| | | UY 35351 A | 29/08/2014 |
| | | WO 01-74143 A1 | 11/10/2001 |
| | | WO 2014-039903 A2 | 13/03/2014 |
| | | WO 2014-039903 A3 | 26/06/2014 |
| US 2017-0189527 A1 | 06/07/2017 | AU 2015-263246 A1 | 17/11/2016 |
| | | AU 2018-222887 A1 | 13/09/2018 |
| | | CA 2947487 A1 | 26/11/2015 |
| | | CA 3050875 A1 | 26/11/2015 |
| | | CN 106659781 A | 10/05/2017 |
| | | CY 1120488 T1 | 10/07/2019 |
| | | DK 3148510 T3 | 16/07/2018 |
| | | EP 2946766 A1 | 25/11/2015 |
| | | EP 2946766 B1 | 02/03/2016 |

| Patent document cited in search report | Publication date | Patent family member | Publication date |
|---|---|---|---|
| | | EP 3148510 A1 | 05/04/2017 |
| | | EP 3148510 B1 | 27/06/2018 |
| | | EP 3403646 A1 | 21/11/2018 |
| | | ES 2572919 T3 | 03/06/2016 |
| | | ES 2683194 T3 | 25/09/2018 |
| | | JP 2017-516847 A | 22/06/2017 |
| | | US 10493152 B2 | 03/12/2019 |
| | | US 2020-0085948 A1 | 19/03/2020 |
| | | WO 2015-177058 A1 | 26/11/2015 |
| WO 2017-136433 A1 | 10/08/2017 | AU 2017-213775 A1 | 16/08/2018 |
| | | CA 3013336 A1 | 10/08/2017 |
| | | CN 109563161 A | 02/04/2019 |
| | | EP 3411401 A1 | 12/12/2018 |
| | | JP 2019-504086 A | 14/02/2019 |
| | | MX 2018009341 A | 15/05/2019 |
| | | US 2019-0030163 A1 | 31/01/2019 |
| EP 2946765 A1 | 25/11/2015 | AU 2015-263340 A1 | 03/11/2016 |
| | | AU 2018-220051 A1 | 06/09/2018 |
| | | CA 2946953 A1 | 26/11/2015 |
| | | CN 106456538 A | 22/02/2017 |
| | | CN 109248315 A | 22/01/2019 |
| | | CY 1120681 T1 | 11/12/2019 |
| | | DK 2946765 T3 | 31/10/2016 |
| | | DK 3145487 T3 | 01/10/2018 |
| | | EP 2946765 B1 | 31/08/2016 |
| | | EP 3050557 A1 | 03/08/2016 |
| | | ES 2600488 T3 | 09/02/2017 |
| | | IL 249115 A | 31/12/2018 |
| | | JP 2017-516846 A | 22/06/2017 |
| | | JP 6334819 B2 | 30/05/2018 |
| | | KR 10-2017-0005864 A | 16/01/2017 |
| | | KR 10-2020-0017553 A | 18/02/2020 |
| | | MX 2016015304 A | 22/02/2017 |
| | | NZ 725360 A | 27/09/2019 |
| | | PL 2946765 T3 | 31/08/2017 |
| | | PL 3145487 T3 | 30/11/2018 |
| | | PT 3145487 T | 19/10/2018 |
| | | SG 10201806300 A | 30/08/2018 |
| | | US 10426832 B2 | 01/10/2019 |
| | | US 2017-0196974 A1 | 13/07/2017 |
| | | US 2020-0016268 A1 | 16/01/2020 |
| | | WO 2015-177057 A1 | 26/11/2015 |
| US 2017-0252437 A1 | 07/09/2017 | BR 112017009021 A2 | 06/02/2018 |
| | | CA 2989930 A1 | 06/05/2016 |
| | | CY 1120937 T1 | 11/12/2019 |
| | | DK 3212667 T3 | 03/12/2018 |
| | | EP 3212667 A1 | 06/09/2017 |

Form PCT/ISA/210 (patent family annex) (January 2015)

INTERNATIONAL SEARCH REPORT
Information on patent family members

International application No.

**PCT/KR2020/000611**

| Patent document cited in search report | Publication date | Patent family member | Publication date |
|---|---|---|---|
| | | EP 3428189 A1 | 16/01/2019 |
| | | ES 2703586 T3 | 11/03/2019 |
| | | HU 1400510 A2 | 30/05/2016 |
| | | LT 3212667 T | 10/12/2018 |
| | | MX 2017005575 A | 01/09/2017 |
| | | PT 3212667 T | 20/12/2018 |
| | | SI 3212667 T1 | 30/11/2018 |
| | | WO 2016-066688 A1 | 06/05/2016 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS,* 331731-18-1